# EUROPEAN PATENT SPECIFICATION

(11) **EP 2 753 716 B1**
(45) Date of publication and mention of the grant of the patent: **01.11.2017**
(21) Application number: 12830701.4
(22) Date of filing: 06.09.2012
(51) Int. Cl.: C12Q 1/68, G01N 33/483, C12Q 1/527, C12Q 1/34

(54) **NOVEL RISK BIOMARKERS FOR LUNG CANCER**
NEUE RISIKOBIOMARKER FÜR LUNGENKREBS
NOUVEAUX BIOMARQUEURS DE RISQUE POUR LE CANCER DU POUMON

(30) Priority: 08.09.2011 US 201161532148 P
(43) Date of publication of application: 16.07.2014
(73) Proprietor: Yeda Research and Development Co. Ltd., 76100 Rehovot (IL)
(72) Inventor: LIVNEH, Zvi, 76100 Rehovot (IL); PAZ-ELIZUR, Tamar, 76100 Rehovot (IL)
(74) Representative: Dennemeyer & Associates S.A.
(86) International application number: PCT/IB2012/054622
(87) International publication number: WO 2013/035071

(56) References cited:
- EP-A1- 2 308 994
- WO-A2-2009/007677
- US-A1- 2004 096 863
- MAREK RUSIN ET AL: "Novel genetic polymorphisms in DNA repair genes: O6-methylguanine-DNA methyltransferase (MGMT) andN-methylpurine-DNA glycosylase (MPG) in lung cancer patients from Poland", HUMAN MUTATION, vol. 14, no. 3, 1 September 1999 (1999-09-01), pages 269-270, XP055177384, ISSN: 1059-7794, DOI: 10.1002/(SICI)1098-1004(1999)14:3<269::AID -HUMU13>3.0.CO;2-6
- MIHI YANG ET AL: "Lack of association between Caucasian lung cancer risk and O6-methylguanine-DNA methyltransferase-codon 178 genetic polymorphism", LUNG CANCER, vol. 44, no. 3, 1 June 2004 (2004-06-01), pages 281-286, XP55177385, ISSN: 0169-5002, DOI: 10.1016/j.lungcan.2003.12.003
- YEN-LI LO ET AL.: 'A Polymorphism in the APE1 Gene Promoter is Associated with Lung Cancer Risk' CANCER EPIDEMIOL BIOMARKERS PREV 2009 vol. 18, no. 1, 05 January 2009, pages 223 - 229, XP055145303
- JUAN LU ET AL.: 'Functional characterization of a promoter polymorphism in APE1/Ref- that contributes to reduced lung cancer susceptibility' FASEB J. vol. 23, no. 10, 23 October 2009, pages 3459 - 3469, XP055145304
- LAURENT GROS ET AL.: 'The major human AP endonuclease (Apel) is involved in the nucleotide incision repair pathway.' NUCLEIC ACIDS RESEARCH vol. 32, no. 1, 02 January 2004, pages 73 - 81, XP055145305
- XIA L ET AL: "Human 3-Methyladenine-DNA Glycosylase: Effect of Sequence Context on Excision, Association with PCNA, and Stimulation by AP Endonuclease", JOURNAL OF MOLECULAR BIOLOGY, ACADEMIC PRESS, UNITED KINGDOM, vol. 346, no. 5, 11 March 2005 (2005-03-11), pages 1259-1274, XP004745230, ISSN: 0022-2836, DOI: 10.1016/J.JMB.2005.01.014
- P. L Lorenzi ET AL: "N-methylpurine DNA glycosylase and 8-oxoguanine DNA glycosylase metabolize the antiviral nucleoside 2-bromo-5,6-dichloro-1-( -D-ribofuranosyl) benzimidazole", Drug Metabolism and Disposition, vol. 34, no. 6, 1 June 2006 (2006-06-01), pages 1070-1077, XP55254262, US ISSN: 0090-9556, DOI: 10.1124/dmd.105.009209

### SEQUENCE LISTING

<110> THE TRUSTEES OF COLUMBIA UNIVERSITY IN THE CITY OF NEW YORK
<120> ISOLATION AND ENRICHMENT OF NUCLEIC ACIDS ON MICROCHIP
<130> 070050.4638
<150> US 61/538,774
   <151> 2011-09-23
<150> US 61/542,124
   <151> 2011-09-30
<150> US 61/588,078
   <151> 2012-01-18
<150> US 61/588,079
   <151> 2012-01-18
<150> US 61/588,082
   <151> 2012-01-18
<150> US 61/590,458
   <151> 2012-01-25
<150> US 61/674,187
   <151> 2012-07-20
<150> US 61/674,191
   <151> 2012-07-20
<150> US 61/674,192
   <151> 2012-07-20
<150> US 61/683,977
   <151> 2012-08-16
<160> 17
<170> PatentIn version 3.5
<210> 1
   <211> 25
   <212> DNA
   <213> Artificial sequence
<220>
   <223> Synthetic oligonucleotide
<220>
   <221> misc_feature
   <222> (1)..(1)
   <223> FAM-Spacer
<400> 1
   gattcaatag gttgtatgca tggtt 25
<210> 2
   <211> 25
   <212> DNA
   <213> Artificial sequence
<220>
   <223> Synthetic oligonucleotide
<220>
   <221> misc_feature
   <222> (1)..(1)
   <223> Double Biotin-Spacer
<400> 2
   ttcaggcaca caaacttgat gggcg 25
<210> 3
   <211> 181
   <212> DNA
   <213> Artificial sequence
<220>
   <223> Synthetic oligonucleotide
<400> 3
   gattcaatag gttgtatgca tggttcatcc gaaccggatt tgagaaactg gaaatcgcca      60
   accccccagt tcactcaagg agcccggccg gatgaacacc cataagcatg cccgattgac      120
   cttcctacgt cgactcgaaa tggtccagca attgatcgcc catcaagttt gtgtgcctga     180
   a                                                                     181
<210> 4
   <211> 80
   <212> DNA
   <213> Artificial sequence
<220>
   <223> Synthetic oligonucleotide
<220>
   <221> misc_feature
   <222> (21)..(60)
   <223> n is a, c, g, or t
<400> 4
   ctacctacga tctgactagc nnnnnnnnnn nnnnnnnnnn nnnnnnnnnn nnnnnnnnnn      60
   gcttactctc atgtagttcc                                                 80
<210> 5
   <211> 20
   <212> DNA
   <213> Artificial sequence
<220>
   <223> Synthetic oligonucleotide
<220>
   <221> misc_feature
   <222> (1)..(1)
   <223> FAM-Spacer
<400> 5
   ctacctacga tctgactagc 20
<210> 6
   <211> 20
   <212> DNA
   <213> Artificial sequence
<220>
   <223> Synthetic oligonucleotide
<220>
   <221> misc_feature
   <222> (1)..(1)
   <223> Dual Biotin-Spacer
<400> 6
   ggaactacat gagagtaagc 20
<210> 7
   <211> 97
   <212> DNA
   <213> Artificial sequence
<220>
   <223> Synthetic oligonucleotide
<220>
   <221> misc_feature
   <222> (25)..(74)
   <223> n is a, c, g, or t
<400> 7
   gcctgttgtg agcctcctgt cgaannnnnn nnnnnnnnnn nnnnnnnnnn nnnnnnnnnn 60
   nnnnnnnnnn nnnnttgagc gtttattctt gtctccc 97
<210> 8
   <211> 78
   <212> DNA
   <213> Artificial sequence
<220>
   <223> Synthetic oligonucleotide
<400> 8
   gcctgttgtg agcctcctgt cgaagcacgt ttatccgtcc ctcctagtgg cgtgcttgag 60
   cgtttattct tgtctccc 78
<210> 9
   <211> 24
   <212> DNA
   <213> Artificial sequence
<220>
   <223> Synthetic oligonucleotide
<400> 9
   gcctgttgtg agcctcctgt cgaa 24
<210> 10
   <211> 23
   <212> DNA
   <213> Artificial sequence
<220>
   <223> Synthetic oligonucleotide
<400> 10
   gggagacaag aataaacgct caa 23
<210> 11
   <211> 87
   <212> DNA
   <213> Artificial sequence
<220>
   <223> Synthetic oligonucelotide
<220>
   <221> misc_feature
   <222> (25)..(64)
   <223> n is a, c, g, or t
<400> 11
   gcctgttgtg agcctcctgt cgaannnnnn nnnnnnnnnn nnnnnnnnnn nnnnnnnnnn 60
   nnnnttgagc gtttattctt gtctccc 87
<210> 12
   <211> 158
   <212> DNA
   <213> Artificial sequence
<220>
   <223> Synthetic oligonucleotide
<400> 12
   cctcaccacc aacttcatcc acgttcacct tgccccacag ggcagtaacg gcagacttct 60
   ccacaggagt cagatgcacc atggtgtctg tttgaggttg ctagtgaaca cagttgtgtc 120
   agaagcaaat gtaagcaata gatggctctg ccctgact 158
<210> 13
   <211> 158
   <212> DNA
   <213> Artificial sequence
<220>
   <223> Synthetic oligonucleotide
<400> 13
   cctcaccacc aacttcatcc acgttcacct tgccccacag ggcagtaacg gcagacttct 60
   cctcaggagt cagatgcacc atggtgtctg tttgaggttg ctagtgaaca cagttgtgtc 120
   agaagcaaat gtaagcaata gatggctctg ccctgact 158
<210> 14
   <211> 20
   <212> DNA
   <213> Artificial sequence
<220>
   <223> Synthetic oligonucleotide
<220>
   <221> misc_feature
   <222> (1)..(1)
   <223> double biotin
<400> 14
   agtcagggca gagccatcta 20
<210> 15
   <211> 20
   <212> DNA
   <213> Artificial sequence
<220>
   <223> Synthetic oligonucleotide
<220>
   <221> misc_feature
   <222> (1)..(1)
   <223> FAM
<400> 15
   cctcaccacc aacttcatcc 20
<210> 16
   <211> 15
   <212> DNA
   <213> Artificial sequence
<220>
   <223> Synthetic oligonucleotide
<400> 16
   acggcagact tctcc 15
<210> 17
   <211> 17
   <212> DNA
   <213> Artificial sequence
<220>
   <223> Synthetic oligonucleotide
<400> 17
   gataggactc atcacca 17

## Description

The present invention, relates to a method of determining a risk of a human subject to develop lung cancer according to claim 1, the use of an assay kit according to claim 13, a method of determining response of a human subject to a lung cancer treatment by a DNA damaging agent according to claim 16, a method of selecting a subpopulation of subjects for a lung cancer diagnostic test according to claim 19.

Lung cancer is the second most prevalent cancer in both men (after prostate cancer) and in women (after breast cancer), accounting for about 15% of all new cases per year, amounting to about 250,000 new cases, and greater than 150,000 deaths per year, in the US alone (American Cancer Society, 2010). The main types of primary lung cancer are small-cell lung cancer (SCLC, about 17%) and non-small-cell lung cancer (NSCLC, about 80%). Of the non-small cell lung cancers, about 40% are adenocarcinoma, 40% squamous cell lung carcinoma, and the remainder carcinoid or of non-specified origin. The most common cause of lung cancer is long-term exposure to tobacco smoke, comprising nearly 90% of non-small cell lung cancer cases. Additional risk factors include second-hand smoke, family history of lung cancer, exposure to radon and other radioactive gases, asbestos, arsenic and air pollution (American Cancer Society, 2010).

Timely diagnosis of lung cancer has been elusive. Symptoms of lung cancer do not usually appear until the disease is already in an advanced stage, and even then may be mistaken for other conditions. Sputum cytology tests, chest X-ray and other diagnostic tools have not been proven effective for screening procedures. However, recent large scale clinical studies have indicated that low-dose CT (spiral CT, LDCT), which can provide a more detailed image of the lungs, is a sensitive and accurate tool for early detection of lung cancer (ELCAP Investigators, N Engl J Med 2006;355:1763-71; NLST team, N Engl J Med. 2011 Aug 4;365(5):395-409).

Due to the enormous expense and complex logistics of LDCT screening of at-risk populations, guidelines for evaluation remain generally unchanged: with most major professional organizations, including the American Cancer Society, not recommending routine lung cancer screening, either for all people or for those at increased risk. However, with increasing evidence of the efficacy of LDCT (studies have indicates that screening with LDCT reduces the chance of dying of lung cancer by 20% in smokers), the demand for implementation of screening protocols will likely increase.

Biomarkers for risk assessment of cancer are useful for early detection and prevention of cancer, and have the potential for use in large-scale screening protocols. Disappointingly, no accurate and sensitive markers for lung cancer have been discovered to date.

DNA repair pathways provide means for rectifying damage, faults and omissions in the replication of the cell's genetic material, including single and double-strand breaks, abasic sites and modifications to nucleic bases. Mismatch repair, nucleotide excision repair and base excision repair (BER) are examples of the three major DNA repair mechanisms.

Correlation of DNA repair capacity with pathologies, specifically cancers, has been undertaken for numerous components of the repair pathways. Autosomal recessive mutations in nucleotide excision repair enzymes result in the UV-hypersensitivity condition known as Xeroderma pigmentosum (XP), with the characteristically high risk of skin and other cancers. Other documented defects of nucleotide excision repair are Cockayne syndrome and trichothiodystrophy.

Mismatch repair defects have been identified in Lynch syndrome, with high incidence of colorectal carcinoma. The BRCA1 and BRCA2 genes, which are markers for hereditary breast cancer, are associated with enzymes of the double strand break DNA repair and homologous recombination repair pathways. Base excision repair abnormalities have been identified in MutYH-associated polyposis (MAP), a hereditary colon cancer predisposition syndrome associated with defects in the repair enzyme MUTYH.

Although some types of cancer can be traced to specific genetic markers, and mutations in DNA repair genes have been identified in lung cancer and kidney tumor cells, correlation of lung cancer with mutations in DNA repair has not yielded accurate and reliable biomarkers suitable for assessing risk (see Vineis et al, J Natl Canc Inst 2009;101:24-36). Many studies investigating global DNA repair capacity via a number of methods (mutagen sensitivity, BPDE-induced DNA adduct assay, gene expression profiling and global DNA repair-host cell reactivation assays) have indicated that individual variability in the DNA repair capacity of humans is correlated with variation in cancer susceptibility, with low overall repair capacity correlated to higher cancer risk for certain types of cancer (see Li et al, Int J Canc 2008; 124:999-1007, and Wang, et al., Clin Canc Res 2010;16:764-74). However, the complexity of the DNA repair pathways and the requirement for manipulation of the cell samples for the assays have often confounded assignment of specific components of the pathways as risk factors. Recently, however, enzymatic activity of 8-oxoguanine DNA glycosylase (OGG1), a DNA base excision repair enzyme responsible for removing oxidized guanines, when measured in non-cancerous tissue samples, was shown to be a valuable risk factor for lung cancer (Paz-Elizur et al. J Natl. Canc. Inst. 2003;95:1312-19) and head and neck cancer (Paz-Elizur et al., Can. Res. 2006; 66: 11683-9).

U.S. Pat. No. 6,358,682 to Jaffee et al. teaches a method, kit and controls for detecting HER-2/neu gene amplification as a predictor of breast cancer recurrence and patient survival.

U.S. Pat. No. 7,097,977 to Takeda et al. teaches a method for measuring sensitivity of a cell to DNA-damaging action by evaluating homologous DNA recombination repair activity.

U.S. Pat. No. 6,773,897 to Herman et al. teaches a method for predicting sensitivity to alkyating chemotherapy agents by determining the methylation state of the gene for MGMT, a DNA repair enzyme.

U.S. Pat. No. 7,288,374 to Pincemail et al teaches evaluation of oxidative stress by assaying a panel of oxidative stress markers in blood cell samples, the markers including DNA repair enzyme expression.

U.S. Pat. Application Pub. No. 2007-0269824 to Albrecht et al. teaches the assessment of DNA damage (adducts, breaks) and/or DNA repair capacity (repair enzyme expression and activity, global repair capacity, damage susceptibility, etc) in a biological sample, in response to stressors, in order to determine a risk of a subject for developing cancer. Baseline results with a small normal population are provided.

U.S. Pat. Application Pub. No. 2004-0096863 to Livneh et al. teaches methods and kits for determining a risk to develop cancer, for evaluating an effectiveness and dosage of cancer therapy and for correlating between an activity of a DNA repair enzyme and a cancer. Correlation between decreased OGG1 catalytic activity in surrogate lymphocytes and risk for lung and head-and-neck cancer was uncovered.

U.S. Pat. Application Pub. Nos. 2003-0104446 and 2006-0147929 to Sauvaigo S. teach the use of "bio-chip" arrays, comprising oligonucleotide or plasmid DNA substrates bearing combinations of various DNA lesions and a variety of substrate sequences, for measuring global DNA repair capacity in a sample. Automation of parts or the whole of the assay is contemplated.

U.S. Pat. Application Pub. No. 20100285456 to Matta teaches measurement of global DNA repair capacity in lymphocytes, using the host reactivation assay, for determining risk of breast cancer.

MAREK RUSIN ET AL discloses "Novel genetic polymorphisms in DNA repair genes: O6-methylguanine-DNA methyltransferase (MGMT) andN-methylpurine-DNA glycosylase (MPG) in lung cancer patients from Poland", HUMAN MUTATION, vol. 14, no. 3, 1 September 1999, pages 269-270.

EP2308994 concerns a method of determining a risk of a subject to develop cancer, the method comprising determining a level of catalytic activity of a DNA repair enzyme in a biological sample of the subject, wherein said DNA repair enzyme is selected from the group consisting of 8-oxoguanine DNA glycosylase (OGG1), AP endonuclease1 (APE1), methylpurine DNA glycosylase (MFG), uracil DNA glycosylase1 (UNG1), uracil DNA glycosylase2 (UNG2), SMUG1, MBD4, mismatch-specific thymine/uracil glycosylase (TDG), enodonuclease III (NTH1), adenine-specific mismatch DNA glycosylase (MYH), 8-oxo-GTPase/8-oxodGTPase (MTH1), dUTPase (DUT), AP endonuclease2 (APE2), deoxyribose phosphate lyase (POLB) and wherein a level of said activity below a predetermined value is indicative of an increased risk of the subject to develop said cancer.

http://dx.doi.org/10.1002/(SICI)1098-1004(1999)14:3<269::AID-HUMU13>3.0.CO;2-6 discloses novel genetic polymorphisms in DNA repair genes: O⁶-methylguanine-DNA methyltransferase (*MGMT*) and *N-*methylpurine-DNA glycosylase (*MPG*) in lung cancer patients from Poland.

MIHI YANG ET AL concerns "Lack of association between Caucasian lung cancer risk and O6-methylguanine-DNA methyltransferase-codon 178 genetic polymorphism", LUNG CANCER, vol. 44, no. 3, 1 June 2004, pages 281-286.

Yen-Li lo et al concerns "a polymorphism in the APE1 gene promoter is associates with Lung cancer risk", cancer epidemiol biomarkers prev 2009, vol.18, no.1, 5 January 2009, pages 223-229.

Juan Lu et al concerns "functional characterization of a promoter polymorphism in APE1/Ref- that contributes to reduced lung cancer susceptibility", FASEB J., vol.23, no. 10, 23 october 2009, pages 3459-3469.

Laurent Gros et al discloses "the major human AP endonuclease (Apel) is involved in the nucleotide incision repair pathway", nucleic acids research , vol.32, no 1, 2 January 2004, pages 73-81.

WO 2009/007677 relates to the use of a DSB repair-inhibitor in the manufacture of a medicament for use in the treatment of a cancer in an individual, wherein the cancer comprises cells which are/is deficient in a BER and/or a SSBR pathway.

### SUMMARY OF THE INVENTION

The present invention relates to a method of determining a risk of a human subject to develop lung cancer, the method comprising determining a level of catalytic activity of N-methylpurine DNA glycosylase (MPG) in a biological sample of the subject, and, according to said level, determining the risk of the subject to develop lung cancer, wherein a level of said catalytic activity of MPG above a first predetermined value is indicative of an increased risk of said subject to develop lung cancer.

Preferably, the method further comprises:
(a) determining a level of catalytic activity of
   (i) apurinic/apyrimidinic endonuclease 1 (APE1) or
   (ii) 8-oxoguanine DNA glycosylase (OGG1) or
   (iii) both apurinic/apyrimidinic endonuclease 1 (APE1) and 8-oxoguanine DNA glycosylase (OGG1)
   in said biological sample, and;
   according to said level, determining the risk of the subject to develop lung cancer, wherein a level of said catalytic activity of MPG above a first predetermined value and either a level of said catalytic activity of APE1 below a second predetermined value or a level of said catalytic activity of OGG1 below a third predetermined value or both a level of said catalytic activity of APE1 below said second predetermined value and a level of said catalytic activity of OGG1 below said third predetermined value
   is indicative of an increased risk of said subject to develop lung cancer.

Preferably, the method further comprises:
determining the level of catalytic activity of at least one of apurinic/apyrimidinic endonuclease 1 (APE1) and 8-oxoguanine DNA glycosylase (OGG1) in said biological sample of the subject;
   (b) determining an integrated DNA repair score for said subject from said level of MPG and at least one of OGG1 and APE1; and
   (c) determining the risk of the subject to develop lung cancer, wherein an integrated DNA repair score below a predetermined value is indicative of an increased risk of said subject to develop lung cancer.

Preferably, said determining said catalytic activity in (a) comprises determining a level of catalytic activity of N-methylpurine DNA glycosylase (MPG) and of apurinic/apyrimidinic endonuclease 1 (APE1) in said biological sample of the subject.

Preferably, said determining said catalytic activity in (a) comprises determining a level of catalytic activity of N-methylpurine DNA glycosylase (MPG) and 8-oxoguanine DNA glycosylase (OGG1) in said biological sample of the subject..

Preferably, said determining said catalytic activity in (a) comprises determining a level of catalytic activity of N-methylpurine DNA glycosylase (MPG), of apurinic/apyrimidinic endonuclease 1 (APE1) and of 8-oxoguanine DNA glycosylase (OGG1) in said biological sample of the subject.

Preferably, said risk or risk level is expressed as an odds ratio (OR) as compared to the risk of developing lung cancer of that of a reference population of normal, apparently healthy individuals matched to said subject or subjects for at least one parameter selected from the group consisting of gender, age, ethnicity and smoking status.

Preferably, the odds ratio for MPG catalytic activity, when determined by the MPG-Hx assay, is 1.18 for each 10 units of catalytic activity or 1.8 for each 1 SD above said predetermined value.

Preferably, the combined odds ratio for MPG and APE1 catalytic activities, relative to that of said reference population, is at least 5, wherein the odds ratio for APE1 is determined by comparing APE1 catalytic activity at the 25^{th} percentile with those of the 75th percentile of control values and the odds ratio for MPG is determined by comparing MPG catalytic activity at the 75th percentile with those of the 25th percentile of control values.

Preferably, the odds ratio is calculated from an integrated DNA repair score for combined MPG, OGG1 and APE1 catalytic activity, wherein when said integrated DNA repair score is below the median of a reference population, said odds ratio is at least 3.0.

Preferably, determining said MPG catalytic activity is effected using a double stranded oligonucleotide substrate having a hypoxanthine lesion (Hx) or using an oligonucleotide substrate having an N6-ethenoadenine lesion (eA), wherein determining said APE1 catalytic activity is effected using an oligonucleotide substrate having a furanyl abasic site lesion (AP) and wherein determining said OGG1 catalytic activity is effected using an oligonucleotide substrate having an 8-oxoguanine lesion.

Preferably, said double stranded oligonucleotide substrate having a hypoxanthine lesion (Hx) comprises an oligonucleotide sequence as set forth in SEQ ID NO: 1 or SEQ ID NO: 7 annealed to an oligonucleotide sequence as set forth in SEQ ID NO: 2 and said oligonucleotide substrate having an N6-ethenoadenine lesion (eA) comprises an oligonucleotide sequence as set forth in SEQ ID NO: 3 annealed to an oligonucleotide sequence as set forth in SEQ ID NO: 4, wherein said oligonucleotide substrate having a furanyl abasic site lesion (AP) comprises an oligonucleotide sequence as set forth in SEQ ID NO: 8 or SEQ ID NO: 10 annealed to an oligonucleotide sequence as set forth in SEQ ID NO: 9 and wherein said oligonucleotide substrate having an 8-oxoguanine lesion an oligonucleotide sequence as set forth in SEQ ID NO: 5 annealed to an oligonucleotide sequence as set forth in SEQ ID NO: 6.

The present invention also relates to the use of an assay kit comprising reagents for determining a level of MPG enzyme activity and at least one of APE1 and OGG1 enzyme activities in a biological sample of said subject for determining a lung cancer risk of said subject.

Preferably, said reagent for determining MPG enzyme activity comprises a double stranded oligonucleotide substrate having a hypoxanthine lesion (Hx) comprising an oligonucleotide sequence as set forth in SEQ ID NO: 1 or SEQ ID NO: 7 annealed to an oligonucleotide sequence as set forth in SEQ ID NO: 2 or an oligonucleotide substrate having an N6-ethenoadenine lesion (eA) comprises an oligonucleotide sequence as set forth in SEQ ID NO: 3 annealed to an oligonucleotide sequence as set forth in SEQ ID NO: 4; wherein said reagent for determining APE1 enzyme activity comprises an oligonucleotide substrate having a furanyl abasic site lesion (AP) comprising an oligonucleotide sequence as set forth in SEQ ID NO: 8 or SEQ ID NO: 10 annealed to an oligonucleotide sequence as set forth in SEQ ID NO: 9 and wherein said reagent for determining OGG1 enzyme activity comprises an oligonucleotide substrate having an 8-oxoguanine lesion comprising an oligonucleotide sequence as set forth in SEQ ID NO: 5 annealed to an oligonucleotide sequence as set forth in SEQ ID NO: 6.

Preferably, said biological sample comprises isolated peripheral blood mononuclear cells.

The present invention relates to a method of determining response of a human subject to a lung cancer treatment by a DNA damaging agent, the method comprising:
(a) determining a level of catalytic activity of N-methylpurine DNA glycosylase (MPG) in a biological sample of the subject, and, according to said level,
(b) determining the appropriate treatment and treatment regimen for treating said subject, wherein a level of said catalytic activity above a predetermined value is indicative of an increased response of said subject to a lung cancer treatment by a DNA damaging agent, and
(c) referring said subject for treatment according to said treatment regimen.

Preferably, the method further comprises determining a level of catalytic activity of apurinic/apyrimidinic endonuclease 1 (APE1) or 8-oxoguanine DNA glycosylase (OGG1) or both APE1 and OGG1 in said biological sample, wherein a level of said catalytic activity of MPG in said sample of the subject above a first predetermined value, and a level of APE1 in said sample of the subject below a second predetermined value or a level of OGG1 in said sample of the subject below a third predetermined value or a level of both APE1 below said second predetermined value and a level of OGG1 below said third predetermined value is indicative of an increased responsiveness of said subject to a lung cancer treatment by a DNA damaging agent.

Preferably, said lung cancer is non-small cell lung cancer.

The present invention also relates to a method of selecting, from within a population of subjects, a subpopulation of subjects for referral for a lung cancer diagnostic test, the method comprising identifying a subpopulation of subjects within said population having an increased risk of developing lung cancer according to the methods of any one of claims 1-12, thereby identifying the subpopulation of subjects for referral for the lung cancer diagnostic test, and referring members of said subpopulation for at least one lung cancer diagnostic test.

Preferably, said lung cancer diagnostic test is selected from the group consisting of mediastinoscopy, bronchoscopy, computerized tomography (CT), spiral (low dose) computerized tomography (LDCT), positron emission tomography (PET), magnetic resonance imaging (MRI), X-ray, sputum cell cytology analysis, lung biopsy, genetic profiling and lung cancer biomarker analysis.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

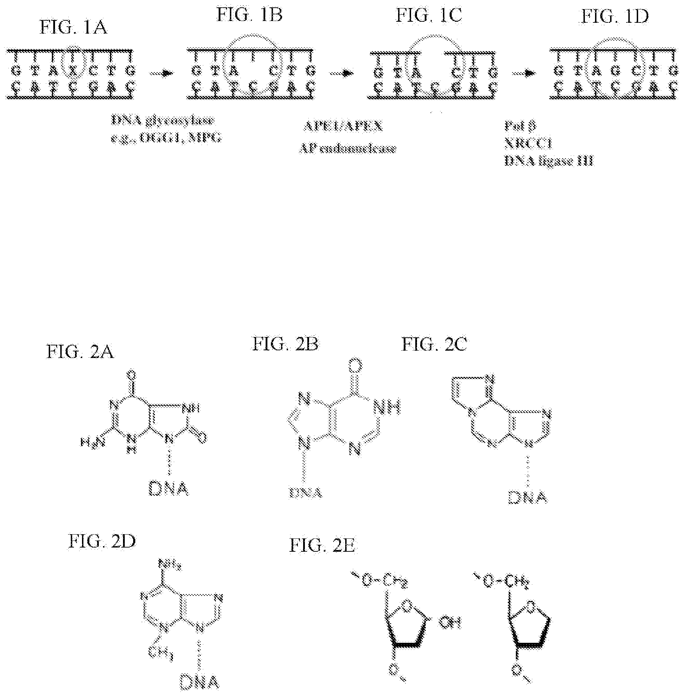

FIGs. 1A-1D are a schematic illustration of the process of Base Excision Repair of DNA. The affected base (X) in damaged DNA (FIG. 1A) is excised by a DNA glycosylase, generating an abasic site (FIG. 1B) (apurinic/apyrimidinic site; AP site). The abasic site is then incised by AP endonuclease 1 (APE1; APEX) (FIG. 1C). Further processing by DNA polymerase β and DNA ligase completes the repair (FIG. 1D);

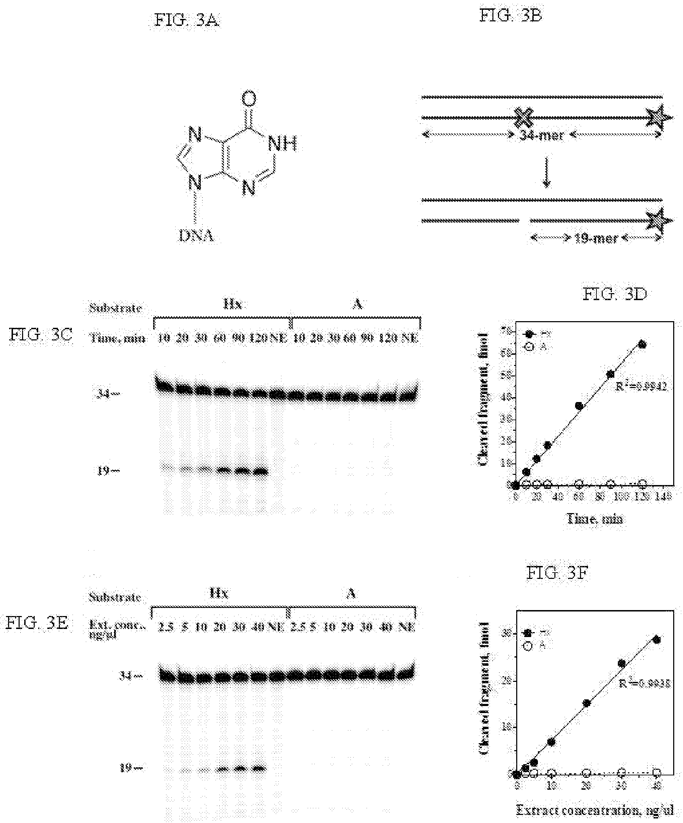

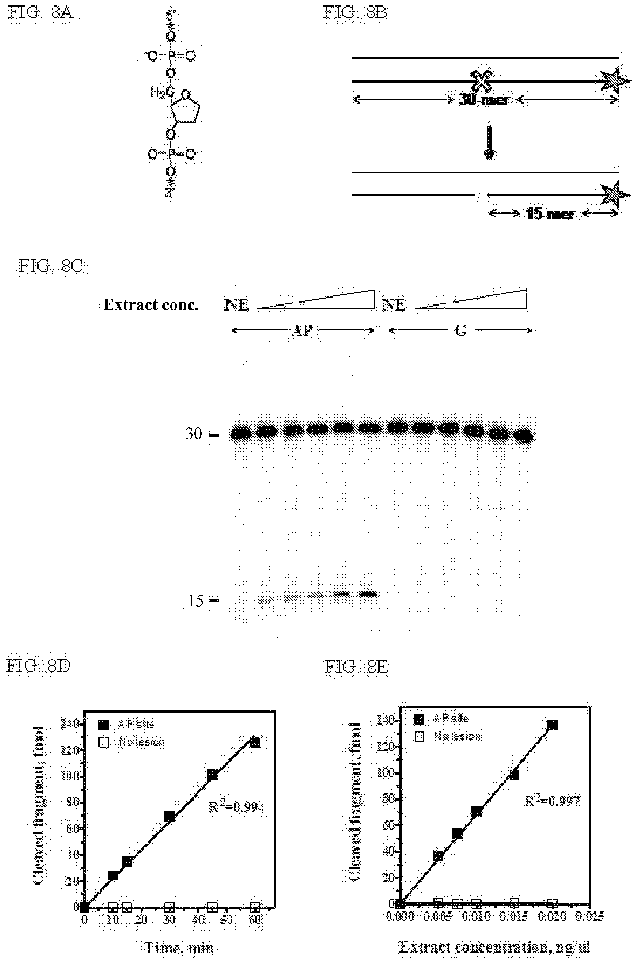

FIGs. 2A- 2E show the chemical structure of DNA lesions repaired by OGG1, MPG(AAG) and APE1. FIG. 2A, 8-oxoguanine (7, 8-dihydro-8-oxoguanine, 8-oxoG); FIG. 2B, hypoxanthine (Hx); FIG. 2C, 1, N6-ethenoadenine (eA); FIG. 2D, 3-methyladenine; FIG. 2E, left: abasic site (also termed apurinic/apyrimidinic site, AP site); FIG. 2E right, furanyl abasic site;
FIGs. 3A- 3F show an assay for MPG (AAG, ANPG) activity on hypoxanthine-containing DNA. FIG. 3A, Structure of hypoxanthine (Hx); FIG. 3B, Schematic of the assay. X marks a site-specific hypoxanthine on a short oligonucleotide. Following incubation with a protein extract, presence of MPG-Hx activity results in the Hx being excised, leaving behind an abasic site, which is cleaved by the APE activity in the extract and subsequent alkali treatment, converting the radiolabeled 34-base oligonucleotide to a 19-base oligonucleotide. Cleavage products are separated by polyacrylamide gel electrophoresis in the presence of urea. Asterisks represent the radiolabeled DNA terminus. FIG. 3C, Time course of MPG-Hx activity in a protein extract prepared from peripheral blood mononuclear cells. The substrate containing Hx lesion (lanes Hx) is cleaved to yield the 19-mer, whereas the control DNA (lanes A) without the damage is not cleaved. FIG. 3D is a graph depicting enzyme activity. The amount of radioactivity in the 19- and 34- base fragments was quantified using phosphorimaging and expressed as a function of the amount of cleaved DNA (in femtomoles) versus time (in minutes). Solid circles (●) indicate DNA containing hypoxanthine; Empty circles (○) indicate control DNA having an A in place of the Hx lesion (see FIG. 3D). FIG. 3E is a photoimage of radioactivity on a denaturing PAGE showing the increase of MPG-Hx activity in increasing amounts of a protein extract prepared from peripheral blood mononuclear cells. The substrate containing Hx lesion (lanes Hx) is cleaved to yield the 19-mer, whereas the control DNA (lanes A) without the damage is not cleaved. FIG. 3F is a graph depicting enzyme activity, using phosphorimaging, expressed as a function of the amount of cleaved DNA (radioactivity) in the 19- and 34-base fragments (in femtomoles) versus the protein concentration (in ng/µl) of the sample. Solid circles (●) indicate DNA containing hypoxanthine; Empty circles (○) indicate control DNA having an A in place of the Hx lesion (see FIG. 3F). The results are from a representative experiment. Note the linearity of the MPG-Hx assay up to 120 minutes and range of protein concentrations from 2.5 ng/µl to 40 ng/µl;

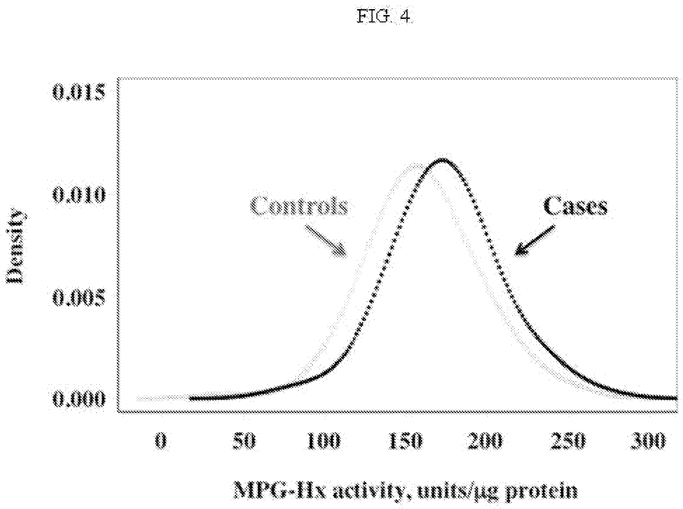

FIG. 4 is a graph illustrating the correlation of MPG-Hx DNA repair activity to lung cancer. MPG-Hx activity, assayed in peripheral blood mononuclear cells as in FIGs. 3A-3F, and expressed as Units/µg protein, in 100 lung cancer patients (black line) and 100 matched controls subjects (grey line). Note the shift to higher MPG-Hx values among the lung cancer patients;

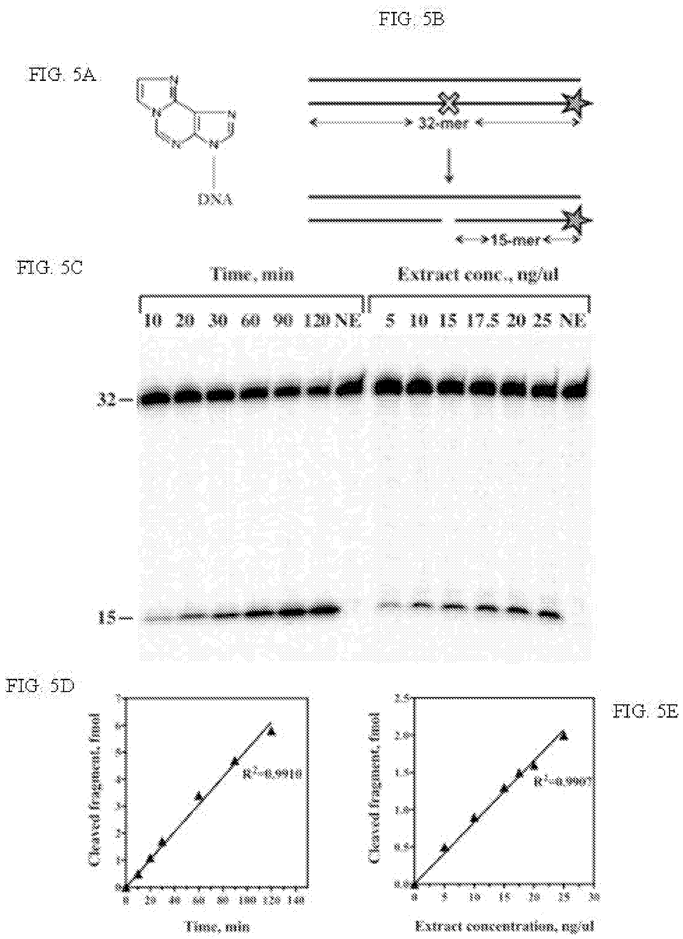

FIGs. 5A-5E show an assay for the MPG catalytic activity using a DNA substrate containing a 1, N6-ethenoadenine lesion. FIG. 5A illustrates the structure of 1, N6-ethenoadenine (eA); FIG. 5B is a schematic outline of the assay. A radiolabeled synthetic short double-stranded DNA carrying a site-specific eA (marked by an X) is incubated with a sample protein extract for MPG determination. MPG-eA activity releases the eA, resulting in an abasic site, which is cleaved by APE present in the sample and subsequent alkali treatment, which in turn converts the radiolabeled 32-base oligonucleotide to a 15-base oligonucleotide. Cleavage products are separated by polyacrylamide gel electrophoresis in the presence of urea. Asterisks represent the radiolabeled DNA terminus. FIG. 5C is a polyacrylamide gel showing a time course and protein titration of MPG-eA activity in a sample prepared from peripheral blood mononuclear cells. The substrate containing the eA lesion is cleaved to yield the 15-mer. The amount of radioactivity in the 15- and 32-base fragments was quantified using phosphorimaging and expressed as a function of the amount of cleaved DNA (in femtomoles) versus time (FIG. 5D) or protein concentration of the sample (FIG. 5E). Note the linear correlation. The results from representative experiments are shown;

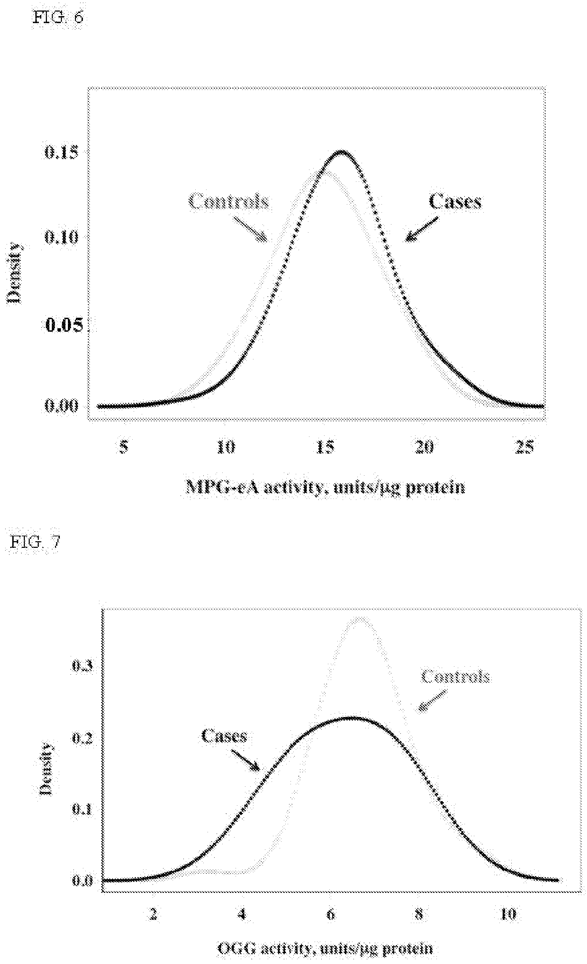

FIG. 6 is a graph illustrating the correlation of MPG DNA repair activity to lung cancer. MPG activity, assayed in peripheral blood mononuclear cells as in FIGs. 5A-5E, and expressed in terms of density, in 100 lung cancer patients (black line) and 100 matched controls subjects (grey line). Note the shift to higher MPG values among the lung cancer patients;
FIG. 7 is a graph illustrating the correlation of OGG-1 DNA repair activity to lung cancer. OGG-1 activity, assayed by fluorescent short DNA substrate (OGG-F) assay in peripheral blood mononuclear cells, and expressed in terms of Units/µg protein, in 100 lung cancer patients (black line) and 100 matched controls subjects (grey line). Note the shift to lower OGG-1 values among the lung cancer patients;
FIGs. 8A-8E show the assay for APE DNA repair activity, using a furanyl abasic site-containing DNA substrate. FIG. 8A illustrates the structure of the furanyl abasic site; FIG. 8B: Schematic outline of the assay. A radiolabeled synthetic short double-stranded DNA carrying a site-specific furanyl abasic site (marked by an X) is incubated with a sample protein extract for APE1 determination. Asterisks represent the radiolabeled DNA terminus. APE activity nicks the substrate, resulting in conversion of the radiolabeled 30-bases to a radiolabeled 15-base oligonucleotide. FIG. 8C is a polyacrylamide gel showing a protein titration of APE1 activity in a protein extract prepared from peripheral blood mononuclear cells, assayed in increasing amounts of the extracts. The substrate containing the furanyl abasic site lesion is cleaved to yield the 15-mer. The amount of radioactivity in the 15- and 30-base fragments was quantified using phosphorimaging and expressed as a function of the amount of cleaved DNA (in femtomoles) versus time (FIG. 8D) or protein concentration of the sample (FIG. 8E). Note the linear correlation. The results from representative experiments are shown;

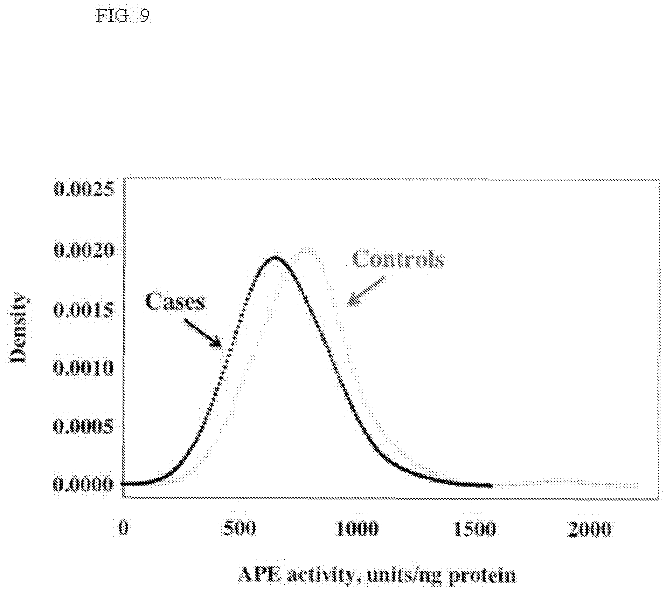

FIG. 9 is a graph illustrating the correlation of APE DNA repair activity to lung cancer. APE-1 activity, assayed in peripheral blood mononuclear cells as in FIGs. 8A-8E, and expressed as Units/ng protein, in 100 lung cancer patients (black line) and 100 matched controls subjects (grey line). Note the shift to lower APE1 values among the lung cancer patients;

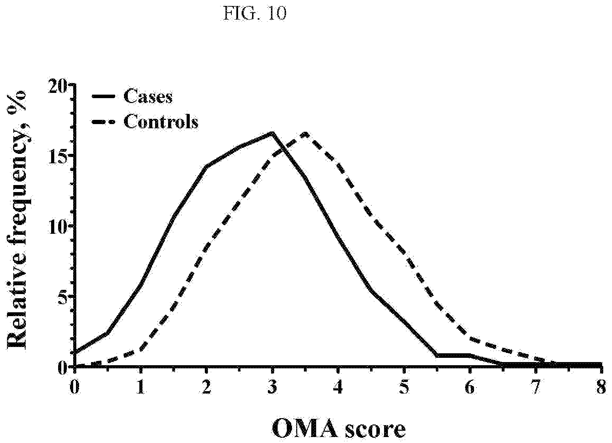

FIG. 10 is a graph illustrating the correlation of integrated DNA repair (OMA) score to lung cancer. OGG1, MPG and APE activity, assayed in peripheral blood mononuclear cells of 100 lung cancer patients (solid line) and 100 matched controls subjects (broken line) as described, was analyzed and combined to provide the integrated DNA repair (OMA) score. Distribution of the integrated DNA repair (OMA) scores in the two populations (according to relative frequency in %) clearly shows a shift to lower integrated DNA repair (OMA) score values among the lung cancer patients.

### DESCRIPTION OF SPECIFIC EMBODIMENTS OF THE INVENTION

The present disclosure, in some embodiments thereof, relates to risk assessment of cancer and, more particularly, but not exclusively, to methods, compositions and kits using biochemical markers for screening subjects for cancer risk. In some embodiments, individuals with increased risk can be referred to further lung cancer diagnostic tests. In yet other embodiments, further lung cancer diagnostic tests are performed on said individuals.

Lung cancer is the second most common form of cancer for both men and women, but the lack of sensitive and accurate methods for risk assessment and early detection confound the effective and efficient deployment of sophisticated diagnostic methods, such as low-dose CT (spiral CT, LDCT), in high risk populations. However, the demand for implementation of screening protocols for LDCT diagnostics will likely increase as LDCT's effectiveness in early detection of lung cancer becomes more widely acknowledged.

Biomarkers for risk assessment of cancer are useful for early detection and prevention of cancer, and have the potential for use in large-scale screening protocols. The present inventors have recently uncovered that reduced enzymatic activity of the DNA repair enzyme 8-oxoguanine DNA glycosylase (OGG1), measured in lymphocytes, is a valuable risk factor for lung cancer (Paz-Elizur et al. J Natl. Canc. Inst. 2003;95:1312-19, Paz-Elizur et al., Can. Res. 2006; 66:11683-9, US2004-0096863).

The present inventors have uncovered that elevated, rather than decreased catalytic activity of another DNA repair enzyme, N-methylguanine DNA glycosylase (MPG) (N-methylpurine DNA glycosylase, DNA 3-methyl adenineglycosylase, MPG; EC 3.2.2.21), when assayed in a biological sample of a subject, is indicative of increased risk for developing lung cancer. MPG activity in lymphocytes of lung cancer patients was consistently greater than that of healthy controls matched for age, gender, area of residence and ethnicity, and adjusted for smoking status.

Thus, according to some embodiments of the present invention, there is provided a method of determining a risk of a human subject to develop lung cancer, the method comprising determining a level of catalytic activity of N-methylpurine DNA glycosylase (MPG) in a biological sample of the subject, and, according to the level, determining the risk of the subject to develop lung cancer, wherein a level of the catalytic activity above a predetermined value is indicative of an increased risk of the subject to develop lung cancer.

The present inventors have also uncovered that catalytic activity of additional DNA repair enzymes are correlated with increased risk for developing lung cancer in a subject. As shown in Examples IV and V herein, reduced levels of APE1 [apurinic-apyrimidinic site endonuclease, APEX1, EC 4.2.99.18], when assayed in a biological sample of a subject, are indicative of increased risk for developing lung cancer. Yet further, the inventors have shown that elevated values of catalytic activity of MPG, combined with reduce catalytic activity of APE1, or OGG1 correlated with even greater indication of risk for lung cancer than any of the individual tests (see Example V herein). Subjects having elevated values of catalytic activity of MPG, combined with reduced catalytic activity of both APE1 and OGG1 correlated with the highest indication of risk for lung cancer.

Thus, according to some embodiments of the present invention, determining the risk of a human subject to develop lung cancer, comprises determining a level of catalytic activity of N-methylpurine DNA glycosylase (MPG) and APE1 in a biological sample of the subject, and, according to the level, determining the risk of the subject to develop lung cancer, wherein a level of the MPG catalytic activity above a first predetermined value and level of APE1 below a second predetermined level is indicative of an increased risk of the subject to develop lung cancer. In some embodiments, determining the risk of a human subject to develop lung cancer, comprises determining a level of catalytic activity of N-methylpurine DNA glycosylase (MPG) and OGG1 in a biological sample of the subject, and, according to the level, determining the risk of the subject to develop lung cancer, wherein a level of the MPG catalytic activity above a first predetermined value and level of OGG1 below a second predetermined level is indicative of an increased risk of the subject to develop lung cancer. In one specific embodiment, determining the risk of a human subject to develop lung cancer, comprises determining a level of catalytic activity of N-methylpurine DNA glycosylase (MPG), APE1 and OGG1 in a biological sample of the subject, and, according to the level, determining the risk of the subject to develop lung cancer, wherein a level of the MPG catalytic activity above a first predetermined value, a level of the APE1 catalytic activity below a second predetermined value and level of OGG1 below a third predetermined level is indicative of an increased risk of the subject to develop lung cancer. Determination of catalytic activity of MPG, APE1 and/or OGG1 is effected on representative biological samples of the subject, and or controls, or can be effected on separate aliquots of the same sample. Methods for assaying MPG, APE1 and OGG1 catalytic activity are detailed hereinbelow.

As used herein, the phrase "predetermined value" refers to a reference level of catalytic activity above (as in the case of MPG) or below (as in the case of OGG1 or APE1) which increased risk of lung cancer can be statistically inferred.

As used herein, the term "risk" refers to the probability of occurrence of a condition or disease, or recurrence of the disease, symptoms, markers or other indicators thereof. Thus, in some embodiments, the risk is a risk of developing lung cancer. In other embodiments, the risk is the risk of recurrence of previously diagnosed lung cancer. In yet other embodiments, the risk can be a risk of developing a specific type of lung cancer. In other embodiments, the risk can be a risk of mortality from the lung cancer, or the probability of survival from the lung cancer. The risk according to the present invention can be expressed in one of a plurality of ways. In one example the risk is expressed as a fold risk increase for developing lung cancer, relative to that of a normal, apparently healthy, population, or a reference control group. In yet other embodiments, the risk is expressed in enzyme specific activity units. In another embodiment, a linear or logarithmic risk scale is generated for either the "fold risk increase" or the "activity units" and the risk is expressed as a magnitude of the scale. In some embodiments, the risk is expressed as an adjusted odds ratio, relative to a selected reference value or range of values of enzyme activity, wherein an odds ratio of 1.0 indicates no difference in risk from that of the reference population, an odds ratio of <1.0 indicates a lesser risk than that of the reference population, and an odds ratio of >1.0 indicates a risk greater than that of the reference population. In other embodiments, the risk is expressed as an adjusted odds ratio, relative to the standard deviation within the range of enzyme activity values of the referenced population. In yet another embodiment, the risk is expressed as an adjusted odds ratio, calculated from an integrated DNA repair score of two or more enzyme activity values. In some embodiments, a predetermined reference value or range is derived from normal healthy individuals. In other embodiments, the reference value or range is derived from individuals who are matched to the subject or subjects according to any one or more parameters including, but not limited to, age, gender, ethnicity, religion, geographical location, smoking status, family history, health history, genetic profile, exposure to carcinogens, occupation, and the like. In certain embodiments, the reference range is adjusted according to one or more parameters including, but not limited to, age, gender, ethnicity, religion, geographical location, smoking status, family history, health history, genetic profile, exposure to carcinogens, occupation, and the like.

The method of the present invention can be used in order to determine responsiveness to a DNA damaging lung cancer treatment, as the use of DNA damaging agents in treatment of cancer is widespread and common. Such agents include, but are not limited to, chemotherapy, radiation, gene therapy and the like.

As used herein the phrase "treatment regimen" refers to a treatment plan that specifies the type of treatment, dosage, schedule and/or duration of a treatment provided to a subject in need thereof (e.g., a subject diagnosed with a pathology). The selected treatment regimen can be an aggressive one which is expected to result in the best clinical outcome (e.g., complete cure of the pathology) or a more moderate one which may relief symptoms of the pathology yet results in incomplete cure of the pathology. It will be appreciated that in certain cases the more aggressive treatment regimen may be associated with some discomfort to the subject or adverse side effects (e.g., a damage to healthy cells or tissue). The type of treatment can include a surgical intervention (e.g., removal of lesion, diseased cells, tissue, or organ), laparoscopy, a cell replacement therapy, an administration of a therapeutic drug (e.g., receptor agonists, antagonists, antibodies, hormones, chemotherapy agents) in a local or a systemic mode, an exposure to radiation therapy using an external source (e.g., external beam) and/or an internal source (e.g., brachytherapy) and/or any combination thereof. The dosage, schedule and duration of treatment can vary, depending on the severity of pathology and the selected type of treatment, and those of skills in the art are capable of adjusting the type of treatment with the dosage, schedule and duration of treatment.

In still other embodiments, the predetermined reference value or range is derived from a previous level or plurality of levels of enzyme activity measured in the subject, prior to a treatment or exposure to risk factor (for example, carcinogens). In yet another embodiment, the subject is a candidate for, or undergoing a lung cancer treatment (e.g. chemotherapy, radiation, surgery and the like) the reference value or range is derived from the subject's pre- treatment, or mid-treatment enzyme level or levels.

In some embodiments, the risk is assigned according to deviance of enzyme levels from median enzyme activity values, for example, median of values from the control reference population. In other embodiments, the risk is assigned according to deviance of enzyme levels from those of reference tertiles, quartiles, quintiles, sextiles, septiles, octiles, noniles, deciles, percentiles or any other division of the referenced enzyme activity values. In still other embodiments, the risk is assigned according to the increments of enzyme activity, for example, increase in risk per 0.1, 0.5, 1.0, 2, 3, 4, 5, 6, 7, 8, 9, 10, 10², 10³, 10⁴ or greater enzyme units over the reference value or range. Thus, in some embodiments of the methods of the present invention, the odds ratio for MPG catalytic activity, when determined by the MPG-Hx assay, is 1.18 for each 10 units of catalytic activity above the predetermined reference value. In other embodiments of the methods of the present invention, the risk is assigned per standard deviation of the distribution of enzyme activity values within the referenced population, the odds ratio for MPG catalytic activity, when determined by the MPG-Hx assay, is 1.8 for each standard deviation.

According to some embodiments, the "odds ratio", or relative risk of an individual or population of individuals to develop lung cancer is determined using conditional logistic regression models with smoking status as an adjusting variable. In some embodiments, smoking status is allotted as either smokers or non-smokers. In other embodiments, smoking status can include current smokers, previous smokers, and never smokers. Additionally and optionally, smoking status can include quantification of amount of tobacco (e.g. numbers of cigarettes, packs, cigars, etc) smoked per day, per year, etc, age of smoking, time since cessation, and the like.

In one embodiment, relative risks estimated for individual enzyme values (e.g. MPG, APE1, OGG) are calculated as a continuous variable (assuming a linear relation with the log odds ratio), a binary variable using the median of the controls as the threshold, and categorized into three groups according to the tertiles of the controls. When categorized according to tertiles, a test for a linear trend in the log "odds ratio" is conducted using scores of 1, 2 and 3 for the three tertile groups.

For pairwise, and three way combinations of MPG tests; OGG and MPG tests; APE and MPG tests; OGG and APE tests and MPG, APE and OGG tests, relative risk can be determined using conditional logistic regression models with smoking status as an adjusting variable. In one embodiment, for the OGG and APE tests, the "odds ratio" is calculated between a person at the 25% percentile versus a person at the 75% percentile of the distribution of assays values among the controls. In another embodiment, for example, for the MPG tests, the odds ratio were calculated between a person at the 75% percentile versus a person at the 25% percentile of the distribution of assays values among the controls.

In another embodiment, the "odds ratio" is calculated according to the value of one standard deviation (SD) of the distribution of observed catalytic activity of each of the enzymes (OGG, MPG, and APE1) within the control samples.

For pairwise or three way combinations of MPG tests; OGG and MPG tests; APE and MPG tests; OGG and APE tests and MPG, APE and OGG tests, relative risk can be expressed as a "combined score", taking into account individuals for whom only one or two values for the two or three enzymes is outside of the normal range. In one embodiment, the integrated DNA repair (OMA) score (OGG, MPG, APE), formulated as the sum of the log odds ratio estimate from logistic regression of (with smoking status in the model), multiplied by the observed value of the sample, e.g. integrated DNA repair (OMA) score = (log odds ratio estimate for OGG X OGG observed value) + (log odds ratio estimate for MPG X MPG observed value) + (log odds ratio estimate for APE X APE observed value). A reduced integrated DNA repair (OMA) score, relative to the control population, is indicative of higher relative risk for the disease.

Thus, in some embodiments, there is provided a method of determining a risk of a human subject to develop lung cancer, the method comprising determining a level of catalytic activity of N-methylpurine DNA glycosylase (MPG) and at least one of apurinic/apyrimidinic endonuclease 1 (APE1) and 8-oxoguanine DNA glycosylase (OGG1) in a biological sample of the subject, determining an integrated DNA repair score for the subject from said level of MPG and at least one of OGG and APE1; and determining the risk of the subject to develop lung cancer, wherein an integrated DNA repair score below a predetermined value is indicative of an increased risk of the subject to develop lung cancer. In some embodiments, the predetermined integrated DNA repair value is an integrated DNA repair value for a reference population of healthy individuals matched for any one or more of age, gender, smoking status, location, ethnicity and the like. Also provided are methods of selecting candidate subjects for a lung cancer diagnostic test based on the integrated DNA repair score, comprising determining an integrated DNA repair score in a biological sample of a subject, and, when the integrated DNA repair score is below a predetermined value, referring the candidate subject for at least one lung cancer diagnostic test. Further, the integrated DNA repair score can be used for selecting a subpopulation of subjects for a lung cancer diagnostic test by collecting a biological sample from each of a population of subjects, determining the integrated DNA repair (from enzyme activity levels of the selected DNA repair enzymes) score in the biological sample of each of the subjects, identifying a sub-population of the subjects having an integrated DNA repair score lower than a predetermined value, and referring the sub-population for at least one lung cancer diagnostic test.

In some embodiments, the combined odds ratio for MPG and OGG1 catalytic activity, relative to that of the reference population, is at least 1.5, at least 2, at least 2.5, at least 3, at least 4, at least 5, at least 7 or more. In some embodiments, the combined odds ratio for MPG and APE1 catalytic activity, relative to that of the reference population, is at least 1.5, at least 2, at least 2.5, at least 3, at least 4, at least 5, at least 7, at least 9, at least 10, at least 12, at least 15, at least 20 or more. In some embodiments, the combined odds ratio for MPG and APE1 and OGG1 catalytic activity, relative to that of the reference population, is at least 1.2, at least 1.3, at least 1.4, at least 1.5, at least 1.6, at least 1.7, at least 21.8, at least 1.9, at least 2.0, at least 2.1, at least 2.2, at least 2.3, at least 2.4 at least 2.5, at least 2.6, at least 2.7, at least 2.8, at least 2.9, at least 3.0, at least 3.1, at least 3.2, at least 3.3, at least 3.4 at least 3.5, at least 3.6, at least 3.7, at least 3.8, at least 3.9, at least 4.0, at least 4.25, at least 4.75, at least 5.0, at least 5.5, at least 6.0, at least 6.5 at least 7.0, at least 8.0, at least 9.0, at least 10, at least 12, at least 15, at least 20, at least 25, at least 30 or more.

In some embodiments, the integrated DNA repair (OMA) score for OGG1, MPG and APE catalytic activity, relative to that of the reference population, is at least 90%, at least 87.5%, at least 85%, at least 82.5%, at least 80%, at least 77.5%, at least 75%, at least 72.5%, at least 70%, at least 67.5%, at least 65%, at least 62.5%, at least 60%, at least 57.5%, at least 55%, at least 52.5%, at least 50%, at least 47.5%, at least 45%, at least 42.5%, at least 40%, at least 37.5%, at least 35%, at least 32.5%, at least 30%, at least 27.5%, at least 25%, at least 22.5%, at least 20%, at least 17.5%, at least 15%, at least 12.5%, at least 10%, at least 7.5% or less than that of the referenced population.

In some specific embodiments, the integrated DNA repair (OMA) score is calculated as 0.00425XAPE + 0.5419XOGG - 0.2541XMPG. In some embodiments, an integrated DNA repair (OMA) score of at least 2.8 or less is indicative of an increased risk of any lung cancer. In other embodiments, an integrated DNA repair (OMA) score of 2.5 or less is indicative of an increased risk of squamous cell carcinoma lung cancer. In still other embodiments, an integrated DNA repair (OMA) score of 2.9 or less is indicative of an increased risk of lung adenocarcinoma.

As used herein, the term "subject" refers to any human being tested by the methods or kits of the present invention. The subject can be a subject who is at risk of having lung cancer [e.g., a genetically predisposed subject, a subject of advanced age, a subject with medical and/or family history of cancer, a subject suffering from COPD, a subject who has been exposed to smoke and/or other carcinogens, occupational hazards, environmental hazards] and/or a subject who exhibits suspicious clinical signs of lung cancer or cancer in general [e.g., persistent cough, hemoptysis, chest pain, shortness of breath, pleural effusion, wheezing, hoarseness, recurrent bronchitis or pneumonia, bone pain, paraneoplastic syndromes, unexplained pain, sweating, unexplained fever, unexplained loss of weight up to anorexia, anemia and/or general weakness]. Additionally or alternatively, the subject can be a healthy human subject undergoing a routine well-being check up or routine screen of a random or representative population. The subject can also be a patient or subject participating in an investigation or test.

Effective early detection of lung cancer can greatly enhance success and reduce the severity of treatment for lung cancer. Recently, sophisticated diagnostic methods such as LDCT have been shown effective for early lung cancer detection, but suffer from a high incidence of false positives (96%), and are prohibitively costly and complex for screening populations. The present invention can be used to effectively select individuals, or groups of individuals, for referral for further diagnostic tests, by assaying the catalytic activity of MPG or APE1 alone, or MPG and either APE1 or OGG1 in pairwise combination, or all three of MPG, APE1 and OGG1 in three way tests combination.

Thus, according to some aspects of some embodiments of the present invention, there is provided a method for selecting candidate subjects for a lung cancer diagnostic test, comprising determining a level of catalytic activity of MPG, or APE1, or MPG and APE1, or MPG and OGG1, or all of MPG and APE1 and OGG1 in a biological sample of the subject. The candidate subject or subjects are referred for at least one lung cancer diagnostic test if the level of MPG catalytic activity is above a predetermined reference value, if the level of APE1 catalytic activity is below a predetermined reference value, or, in combined assays, if the level of MPG catalytic activity is above a predetermined reference value and the level of APE1 and/or OGG1 catalytic activity is below a predetermined reference value. In other embodiments, the selecting is affected by determining a level of catalytic activity of MPG and at least one of OGG1 and APE1 in the samples, determining an integrated DNA repair score for the subject, and referring the subject for further diagnosis if the integrated DNA repair score is lower than a predetermined value.

The methods of the present invention can be used for selecting candidate subpopulations of subjects from a population, for referral to lung cancer diagnostic test or tests. In some embodiments, the population is a random population defined, for example, according to geographical proximity (e.g. presence in or around a center or facility for lung cancer screening), or a sample population randomly selected for screening from among a population of employees, students, university faculty, etc, or the population may be a population defined by specific parameters, including, but not limited to age, gender, ethnicity, and the like, or, optionally or additionally, including lung cancer risk factors, [e.g., genetical predisposition, advanced age, medical and/or family history of cancer, COPD, exposure to smoke and/or other carcinogens, occupational hazards, environmental hazards] and/or a population comprising subjects exhibiting suspicious clinical signs of lung cancer or cancer in general. Thus, according to some embodiments of the present invention there is provided a method of selecting a subpopulation of subjects for a lung cancer diagnostic test, comprising determining a level of catalytic activity of MPG or APE1 or both, or MPG and OGG1 or all of MPG and APE1 and OGG1 in a biological sample from each of the subjects, identifying a subpopulation of subjects having a level of MPG catalytic activity higher than a predetermined value, and/or a level of APE1 or OGG1 lower than a predetermined value in the samples, and referring the subpopulation for at least one lung cancer diagnostic test. In certain embodiments, the method further includes collecting a biological sample from each of the population of subjects. In other embodiments, the selecting is affected by determining a level of catalytic activity of MPG and at least one of OGG1 and APE1 in each of the samples, determining an integrated DNA repair score for each of the subjects, and identifying a subpopulation having an integrated DNA repair score lower than a predetermined value.

Candidate subject or subjects, or candidate subpopulations can be referred, for example, to repeat the measurements of catalytic activity according to the present invention at one or more later times, or to undergo additional, specific tests such as mediastinoscopy, bronchoscopy, computerized tomography (CT), spiral (low dose) computerized tomography (LDCT), positron emission tomography (PET), magnetic resonance imaging (MRI), X-ray, sputum cell cytology analysis, lung biopsy, genetic profiling and lung cancer biomarker analysis. In certain embodiments, the additional lung cancer diagnostic test is LDCT.

In certain embodiments, subjects or sub-populations which have increased risk for developing lung cancer, as determined by the methods employing assay of DNA repair enzymes of the present invention, can be counseled to alter their life style and/or behavior to reduce exposure to lung cancer risk factors, for example, reduction or cessation of smoking, reduction or elimination of exposure to radiation, reduction or elimination of exposure to particulate pollution (talc, asbestos) and the like.

One potential role of the method of the present invention in a clinical setting in which highly sensitive and specific techniques for imaging lung cancer, such as spiral CT (LDCT) scanning are available is as a complement to such scanning. Briefly, high-risk or randomly chosen patients could potentially be screened for lung cancer in a rational and cost-effective program, as follows: primary assaying of MPG, APE1, OGG1 or combinations of DNA repair enzyme activity levels according to the methods of the present invention (low cost); if the results are positive, go to secondary screening with spiral CT scan of the chest (intermediate cost); if that has a positive result, go to final testing with bronchoscopy and biopsy (high cost). For example, it would be straightforward to implement a DNA repair enzyme activity level according to the present invention as a screening program for at-risk populations because each site would require only blood or other biological sample collection apparatus. Samples can then be sent to a central laboratory for assay of enzyme activity.

In one embodiment of some aspects of the present invention, the subject is a present smoker, has a history of smoking (former smoker), or has never smoked (never smoker), has been exposed to (inhaled) second-hand smoke, has been exposed to (inhaled) asbestos, airborne particulates (e.g. talc) or has been exposed to (inhaled) carcinogens. In other embodiments of the present invention, the subject has been exposed to ionizing radiation, radon or other radioactive gas, and the like.

As used herein, the term "lung cancer" refers to any cancerous growth in the lung. In some embodiments, the lung cancer is small cell lung cancer (SCLC), and non-small cell lung cancer (NSCLC), characterized by the cell size when viewed under the microscope. In other embodiments, primary NSCLC comprises mostly adenocarcinoma (including bronchoalveolar cell carcinoma), squamous cell carcinoma and large cell carcinoma. As used herein, the term lung cancer also includes lung cancers of rare cell types, such as carcinoid tumors and lymphoma. In some embodiments, a lung cancer patient is a patient diagnosed with lung cancer on the basis of imaging, biopsy, staging, etc.

The present inventors have uncovered that increased MPG catalytic activity is correlated to increased risk of developing lung cancer. Thus, according to some aspects of certain embodiments of the present invention, the method comprises determining a level of MPG catalytic activity in a biological sample of a subject. As used herein, the phrase "biological sample" refers to, but is not limited to a sample of a tissue, a cell or cells, fluid of the subject and the like. In some embodiments, the biological sample is a scraped cell sample, a blood sample, or a biopsy sample. In some embodiments, the biological sample is a blood sample, for example, a whole blood sample, a blood cell sample or a serum sample. In still other embodiments, the sample is a peripheral blood cell sample. As used herein, the phrase "peripheral blood cell sample" refers to a sample taken from circulating blood as opposed to blood cells within the lymphatic system, spleen, liver, or bone marrow. Peripheral blood comprises erythrocytes, leukocytes and platelets. In some embodiments, the biological sample comprises isolated blood cells, for example, isolated mononuclear cells. In some embodiments, the peripheral blood sample is a peripheral blood mononuclear cell sample, prepared from whole peripheral blood.

Peripheral blood cell samples are typically taken with a syringe with a needle, and/or with an evacuated container (e.g. Vacutainer ®) and a needle. Samples may also be taken from blood collection bags.

Methods of processing peripheral blood cell samples are known in the art and further described in the Examples section herein below.

In some embodiments of the present invention, catalytic activity is determined from a fresh biological sample. As used herein, the term "fresh" refers to a sample which has not been preserved prior to assay of catalytic activity. In some embodiments, the fresh sample is assayed <1 hour from its removal from the subject. In yet another embodiment, the fresh sample is assayed about 1 hour, about 2 hours, about 3, about 4, about 5, about 6, about 8, about 10, about 12 about 18, about 24 hours, about 1 day, about 2 days, about 3 days or more from removal from the subject. In other embodiments of the present invention, the sample is a processed sample. As used herein, the phrase "processed sample" refers to a sample which has been treated, after removal from the subject, in order to isolate, purify, alter and/or preserve a component or components of the sample. For example, in some aspects of some embodiments of the present invention, the sample is an extract of peripheral blood cells. In yet further embodiments, catalytic activity is determined in a whole cell extract or protein extract of the peripheral blood cells, for example, in a protein extract of peripheral blood mononuclear cells, as further described in the Examples section herein.

In some aspects of embodiments of the present invention, the sample is a preserved sample, such as, but not limited to, a refrigerated sample or a cryopreserved protein extract of peripheral blood mononuclear cells. Methods for preservation of samples, without significant deterioration in their enzyme activity are known in the art, for example, as described in detail in the Examples section herein.

According to some aspects of embodiments of the present invention, enzyme activity of DNA repair enzymes can be assayed using any one or more of oligo- or polynucleotide substrates bearing a lesion recognized by the DNA repair enzyme. In some aspects of some embodiments of the present invention, the enzyme is a DNA glycosylase, which removes an single altered base from the sugar backbone, such as methylpurine DNA glycosylase (N-methylpurine DNA glycosylase, DNA 3-methyl adenineglycosylase II, MPG; EC 3.2.2.21) or 8-oxoguanine DNA glycosylase (OGG 1; EC 3.2.2-) and the substrate is an oligonucleotide or polynucleotide with at least one altered base. Exemplary substrates for MPG include, but are not limited to, double stranded DNA with at least one strand bearing a hypoxanthine lesion (Hx, see Figure 2B, 3A), for example, SEQ ID NO: 1, or bearing a 1, N6-ethenoadenine lesion (eA, see Figure 2C, 5A), for example, SEQ ID NO: 3. Exemplary substrates for OGG1 include, but are not limited to, double stranded DNA with at least one strand bearing a 8oxoguanine lesion (8oxog, see Figure 2A), for example, SEQ ID NO: 11(OGG1 substrate).

In other aspects of some embodiments of the present invention, the enzyme is an AP endonuclease, which hydrolyses the phosphodiester bond at the baseless sugar, such as APE1 [apurinic-apyrimidinic (AP) site endonuclease, APEX1, EC 4.2.99.18] and the substrate is an oligonucleotide or polynucleotide with at least one abasic site. Exemplary substrates for APE1 include, but are not limited to, double stranded DNA with at least one strand bearing a native abasic site, or a modified abasic site, such as furanyl abasic site (see Figure 2E and 8A), for example, SEQ ID NO: 8).

Methods for preparation of oligonucleotide substrates for assaying DNA repair enzymes are well known in the art. Oligonucleotides designed according to the teachings of the present invention can be generated according to any oligonucleotide synthesis method known in the art such as enzymatic synthesis or solid phase synthesis. Equipment and reagents for executing solid-phase synthesis are commercially available from, for example, Applied Biosystems. Any other means for such synthesis may also be employed; the actual synthesis of the oligonucleotides is well within the capabilities of one skilled in the art and can be accomplished via established methodologies as detailed in, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988) and "Oligonucleotide Synthesis" Gait, M. J., ed. (1984) utilizing solid phase chemistry, e.g. cyanoethyl phosphoramidite followed by deprotection, desalting and purification by for example, an automated trityl-on method or HPLC.

The DNA substrate can be a short section of double stranded DNA, comprising about 5, about 10, about 15, about 20, about 25, about 30, about 32, about 35, about 37, about 39, about 40, about 45, about 50, about 75, about 100 or more base pairs and at least one of the lesions recognized by the enzyme activity to be assayed. In one particular embodiment the DNA substrates bear single lesions and are 30-40 base pairs in length. Alternatively, or additionally, the DNA substrate can be a large section of DNA, such as a plasmid, comprising thousands or more base pairs and bearing the substrate lesion(s). DNA substrates bearing multiple lesions include, but are not limited to, substrates having multiple lesions of identical type (for example, Hx or εA or 8oxoG), or complex substrates having one or more lesions of least two different types (for example, Hx and eA, or Hx and 8oxoG, or eA and 8oxoG).

A substrate of the invention can thus have at least one lesion of at least one type or at least one lesion of at least two types (complex substrate), the lesions preferably being positioned at predetermined site(s) in the DNA substrate. The lesion(s) for MPG substrate can be of any type, including, but not limited to, 3-methyladenine, 7-methyladenine, 3-methylguanine, 7-methylguanine, hypoxanthine, 1, N6-ethenoadenine and 1,N2-ethenoguanine. Lesions for APE1 assay include any abasic site, including but not limited to furanyl abasic site. Lesions for OGG1 assay include, but are not limited to 2,5-amino-5-formamidopyrimidine and 7,8-dihydro-8-oxoguanine.

A lesion can be introduced at a unique and defined location (site) in a DNA molecule using solid phase DNA synthesis, using in sequence the four conventional phosphoramidite building blocks used in the synthesis of oligodeoxynucleotides and additional at least one modified phosphoramidite building block, which when introduced into the DNA introduces a lesion therein, which lesion is recognizable by a DNA repair enzyme. In the alternative, a DNA molecule is exposed to a mutagenic agent (e.g., an oxidative agent or UV radiation) which forms one or more lesion of one or more types therein. Even when using this method, one can select a presubstrate which will result in a product (substrate of the invention) in which the lesions are non-randomly distributed, since the extent by which a specific lesion is formed in DNA is often dependent on the DNA sequence.

Other alternatives also exist. For example, one can oxidize a plasmid DNA with an oxidizing agent. This will form several lesions in the plasmid DNA. One can now use this plasmid DNA to assay a repair enzyme that acts on this DNA, without knowing precisely where the lesions are. The enzyme will produce a nick in the DNA, and this will convert the plasmid from the supercoiled closed form to the nicked (open circular) form. These two can be easily distinguished by gel electrophoresis or gradient centrifugation. In another example a sequence of DNA is enzymatically synthesized in the presence of lesioned building blocks. Other alternatives are also known, such as chemical deamination, etc.

Detection of DNA glycosylase or AP endonuclease activity can be effected, for example, by monitoring the creation of abasic sites, or by monitoring the sensitivity of the abasic DNA to breakage (for example, nicking by alkali treatment), or by monitoring the nicking activity of the AP endonucleases, converting the substrates to shorter oligonucleotide products. As detailed in the Examples section below, for example, detection of DNA repair enzyme activity can be effected by employing a DNA substrate of defined length, bearing a desired lesion at a predetermined site (see Figures 3B, 5B and 8B), and detection of breakage by monitoring the appearance of breakage products (fragments) of expected length (see Figures 3C, 5C and 8C).

Detection of enzyme activity can be effected by detection of presence of conversion products, for example, fragments of specific length, sequence, properties and the like. Various methods and devices for detection of DNA repair enzyme activity are well known in the art, and are detailed, for example, in U.S. Pat. Application Pub. No. 2007-0269824 to Albrecht et al. and U.S. Pat. Application Pub. Nos. 2003-0104446 and 2006-0147929 to Sauvaigo S.

According to some embodiments, the substrate DNA is labeled and monitoring of the conversion of substrate DNA to products is effected by detection of the label in specific sized DNA fragments. The oligonucleotide or polynucleotide substrates used by the present invention can be labeled either directly or indirectly using a tag or label molecule. Such labels can be, for example, fluorescent molecules (e.g., Yakima Yellow, fluorescein or Texas Red), radioactive molecule (e.g., ³²P-γ-ATP or ³²P-α-ATP) and chromogenic substrates [e.g., Fast Red, BCIP/INT, available from (ABCAM, Cambridge, MA)]. Direct labeling can be achieved by covalently conjugating a label molecule to the substrate (e.g., using solid-phase synthesis) or by incorporation via polymerization (e.g., in a T4 polynucleotide kinase reaction, using an *in vitro* DNA synthesis reaction or random-primed labeling). Indirect labeling can be achieved by covalently conjugating or incorporating to the substrate a non-labeled tag molecule (e.g., Digoxigenin or biotin) and subsequently subjecting it to a labeled molecule (e.g., anti-Digoxigenin antibody or streptavidin) capable of specifically recognizing the non-labeled tag.

It will be appreciated that the marker or label moiety should be a marker or label affording efficient, specific and cost -effective detection, with acceptable levels of safety in handling, but also devoid of artifact-producing alterations of the substrate. In Examples II-VI of the Examples section below, DNA repair enzyme assays using fluorescent (e.g. Yakima Yellow) tagged substrates (e.g. SEQ ID NO: 7 and SEQ ID NO: 10) were shown as accurate and reproducible as assays with radioactive labeled oligonucleotide substrates. Thus, in one embodiment of the present invention, determining the DNA repair activity is effected using a radiolabeled and/or fluorescent-tagged oligonucleotide substrate. In specific embodiments the DNA repair enzyme activity is determined using the fluorescent substrates SEQ ID NO: 7 (MPG), SEQ ID NO: 10 (APE1) and SEQ ID NO: 5 (OGG1), or radiation-tagged substrates SEQ ID NOs: 1 or 3 (MPG) or 8 (APE1).

Reaction products can be detected variety of DNA detection methods such as Southern blot analysis, PCR, fluorometry, sequencing and the like. In specific embodiments, detection of radioactive DNA reaction products is effected following denaturation, by denaturing (e.g. urea) PAGE and visualization of the denatured fragments by phosphorimagery, as in Examples II-V. In some embodiments, the detection of fluorescent reaction products is effected by automated monitoring of the fluorescence of the denatured fragments of the reaction mixture using capillary gel electrophoresis, for example, on the ABI3130XL genetic analyzer (Applied Biosystems, Foster City, CA).

Alternately and optionally, the DNA repair enzyme protein can be quantitated in the sample, or sample extract. Quantitation of the presence of DNA repair enzyme in the sample can be effected via specific antibodies (polyclonal and/or monoclonal) using immunological techniques such as Western blotting, ELISA, and the like.

According to an additional aspect of the present invention there is provided an assay kit for determining a risk of a human subject to develop lung cancer, comprising reagents for determining a level of at least one of MPG and/or APE1 activity of a DNA repair/damage preventing enzyme in a biological sample of a subject. In certain embodiments, the kit includes, a package including, contained in sealable containers, a DNA substrate for MPG and/or APE1 having at least one lesion therein and a reaction buffer selected suitable for supporting DNA repair activity. Optionally and additionally, the kit comprises a DNA substrate for OGG1. In specific embodiments, the DNA substrates comprise, for example, SEQ ID NOs: 1, 7 and 2, or 3 and 4, for assaying MPG, SEQ ID NOs: 8, 9 and 10 for assaying APE1, and SEQ ID NOs. 5 and 6 for assaying OGG1. In certain embodiments, the DNA substrates are ds DNA comprising the abovementioned substrates annealed in pairs, e.g. SEQ ID NOs: 1+2, 3+4, 5 (or 11)+6, 7+2, 8+9 and 10+9.

In a specific embodiment, the kit may also include containers for collecting samples, for example, samples of tissue scrapings, blood samples, or biopsies, and may further contain preservatives, e.g. protease inhibitors. The kit may also include test tubes for separating blood cells, for example, lymphocytes, PBMC, etc. In some embodiments, the test tubes are prepackaged with an anti-coagulant, such as, but not limited to, heparin, ACD or CPDA-1. In some embodiments, the kit further includes a liquid having a specific gravity selected effective in separating lymphocytes from red blood cells via centrifugation, e.g., Ficoll contained in lymphocytes isolation tubes. In specific embodiments, the kit includes a solution having osmolarity selected effective in lysing red blood cells.

In some embodiments the kit includes instructions for use in a method of determining a lung cancer risk in a subject. Such instructions may include, but are not limited to, ranges of reference values for evaluating results of the assays of DNA repair enzyme activity in the samples, or tables for assigning specific reference value ranges according to categories such as gender, age, smoking status and the like. Instructions may further include protocols for alerting physicians to the results, for referring the subject, or subjects to further diagnostic tests and/or treatment, such as, but not limited to, LDCT, biopsy, sputum cytology tests and the like, and suggestions for altering the subject's life style and behavior to reduce exposure to lung cancer risk factors, for example, reduction or cessation of smoking. The instructions can be provided in a paper format, or can be accessible by computer, e.g. on CD-ROM and/or other computer readable media. Such instructions can also provide access to relevant statistical databases.

The kit may also be accompanied by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for diagnostic tests or of an approved product insert. Containers comprising the reagents for effecting the method of the invention may also be prepared, and labeled for diagnosis of lung cancer risk, or selection of individuals for further lung cancer diagnostic tests, as is further detailed above.

As used herein the term "about" refers to ± 10%.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

Various embodiments and aspects of the present invention as delineated hereinabove and as claimed in the claims section below find experimental support in the following examples.

### REFERENCE EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1, 2, 317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader.

### Materials and Experimental Methods

***Study participants:*** Patients were accrued from one of the hospitals in a defined area of Northern Israel. Controls were insurees of an HMO identified from the same geographical area. All study participants (patients and controls) had similar basic health insurance plans and access to health services. Controls were individually matched to the patients by sex, year of birth, residence (defined by primary clinic location), and ethnic group (Jewish, Arab). Controls were selected from the enrollee list of the HMO from which multiple matched candidates were available and one was randomly assigned as a control. Cases and controls were excluded only if they had a former diagnosis of lung cancer. Participants provided written informed consent at time of enrollment and were interviewed in-person to obtain information about their personal and family history of cancer, detailed active and passive smoking history. Diagnoses of lung cancer were made independently by the diagnosing hospitals and included information on histological type, TNM staging and tumor grade. All procedures were reviewed and approved by a hospital institutional review board. Cases and their matched healthy controls were selected based on the availability of a blood sample drawn prior to the operative procedure or any treatment intervention (chemotherapy or radiotherapy) and was immediately shipped to the laboratory for processing.

***Isolation of peripheral lymphocytes:*** The blood samples were processed 18-24 hours after collection. A 100 µl aliquot from each sample of whole blood was analyzed using a Cobas Micros (Roche Diagnostic System) blood counter. The whole blood sample was centrifuged at 400xg for 10 minutes at 20 °C, and plasma rich with contaminating platelets was removed. PBS (Dulbecco's phosphate buffered saline, Sigma) supplemented with 2 mM EDTA was added to the remaining whole blood portion to a final volume of 30 ml (when using 10 ml CPDA-1 anticoagulant collection tubes) or 35 ml (when using ACD anticoagulant evacuated collection tubes), and peripheral blood mononuclear cells (PBMC) were isolated by density gradient centrifugation of the diluted whole blood on a polysucrose-sodium metrizoate medium in a UNI-SEP tube (NOVA*med*, Jerusalem, Israel) at 1,000 x g for 30 minutes at 20 °C.

Following centrifugation the PBMC fraction was rinsed with PBS buffer + 2 mM EDTA. Red blood cells were eliminated by lysis in 5 ml of 155 mM NH₄Cl; 10 mM KHCO₃; 0.1 mM EDTA for 4 minutes at room-temperature. The lymphocytes were washed with PBS + 2mM EDTA, and suspended in 1 ml PBS. The number of white blood cells in this suspension was determined using a Cobas Micros (Roche Diagnostic System) blood cell counter.

Samples containing 2.5-10 x 10⁶ cells were precipitated by centrifugation at 5,000 rpm, for 4 minutes at room temperature. The pellet was then resuspended to a concentration of 50,000 cells/µl in 50 mM Tris·HCl (pH 7.1), 1 mM EDTA, 0.5 mM spermidine, 0.1 mM spermine, and a protease inhibitor cocktail (Sigma). The cells were incubated on ice for 30 minutes, after which they were frozen in liquid nitrogen. The frozen lymphocytes were stored at -80 °C.

***Reparation of a protein extract:*** The frozen lymphocytes were thawed at 30 °C, after which their protein content was extracted with 220 mM KCl, for 30 minutes on ice. Cell debris was removed by centrifugation at 13,200 rpm for 15 minutes at 4 °C. Glycerol was added to the protein extract to a final concentration of 10%, and the extract was frozen in liquid nitrogen. Determination of protein concentration was adapted to a robotic platform using the BCA assay kit (Pierce) and bovine γ-globulin as a standard. Liquid handling was performed by Freedom EVO 200 robot (Tecan) and 562 nM absorbance was measured by Infinite M200 plate reader (Tecan).

***DNA substrates:*** The DNA substrates were prepared by annealing two complementary synthetic oligonucleotides as detailed below.

***Radioactive DNA substrates:*** The oligonucleotide containing the site-specific lesion was 5' ³²P-labeled using γ-³²P ATP and T4 polynucleotide kinase, and annealed to the complementary oligonucleotide. The radiolabeled duplex was purified by polyacrylamide gel electrophoresis (PAGE) on a native 10 % gel. Its concentration was determined by NanoDrop® ND-1000 Spectrophotometer.

***Fluorescent DNA substrates:*** The oligonucleotide containing the site-specific lesion was 3' labeled with a Yakima Yellow fluorophore (Glen 205921), and annealed to the complementary oligonucleotide. The fluorescent duplex was purified by PAGE on a native 10 % gel. Its concentration was determined by NanoDrop® ND-1000 Spectrophotometer.

***Oligonucleotides:*** Oligonucleotides were synthesized using an Expedite 8909 DNA Synthesizer (Applied Biosystems, Foster City, CA) and the special lesion building blocks were purchase from Glen Research (Sterling, VA). Oligonucleotides were also purchased from several commercial sources including Sigma, IDT (Coralville, Iowa), Proligo (Boulder, CO) and Metabion (Martinsried, GmBH). Below are the oligonucleotides used for each of the DNA repair assays:
***MPG-Hx assay:*** 34-mer oligonucleotide containing hypoxanthine (Figure 2B) had the sequence 5'- GT CCG GTG CAT GAC ACT GTX ACC TAT CCT CAG CG -3' (SEQ ID NO:1) (X = hypoxanthine). The complementary oligonucleotide had the sequence 5'- CG CTG AGG ATA GGT TAC AGT GTC ATG CAC CGG AC -3' (SEQ ID NO:2).
***MPG***-***eA assay:*** 32-mer oligonucleotide containing 1, N6-ethenoadenine (eA) (Figure 2C) had the sequence 5'- CCT ACC TAG CGA CCT XCG ACT GTC CCA CTG CT -3' (SEQ ID NO:3) (X = eA). The complementary oligonucleotide had the sequence 5'- AGC AGT GGG ACA GTC GTA GGT CGC TAG GTA GG -3' (SEQ ID NO:4).
***OGG-F assay:*** 32-mer oligonucleotide containing 8-oxoguanine (Figure 2A) and 3' Yakima yellow fluorescent tag had the sequence 5'- CCG GTG CAT GAC ACT GTX ACC TAT CCT CAG CG -3' (SEQ ID NO:5) (-YY) (X = 8-oxoguanine; YY = Yakima yellow tag). The complementary oligonucleotide had the sequence 5'-CGC TGA GGA TAG GTC ACA GTG TCA TGC ACC GG -3' (SEQ ID NO: 6).
***MPG-Hx-F assay:*** 34-mer oligonucleotide containing hypoxanthine (Figure 2B) and 3' Yakima yellow fluorescent tag had the sequence 5'- GT CCG GTG CAT GAC ACT GTX ACC TAT CCT CAG CG (SEQ ID NO: 7) (-YY) -3' (X = hypoxanthine; YY = Yakima yellow tag). The complementary oligonucleotide had the sequence 5'- CG CTG AGG ATA GGT TAC AGT GTC ATG CAC CGG AC -3' (SEQ ID NO:2).
***APE assay:*** 30-mer oligonucleotide containing furanyl abasic site (Figure 2E) had the sequence 5'- G GTG CAT GAC ACT GTX ACC TAT CCT CAG CG -3' (SEQ ID NO:8) (X = furanyl abasic site). The complementary oligonucleotide had the sequence 5'- CG CTG AGG ATA GGT CAC AGT GTC ATG CAC C -3' (SEQ ID NO:9).
***APE-F assay:*** 30-mer oligonucleotide containing furanyl abasic site (Figure 2E) and 3' Yakima yellow fluorescent tag had the sequence 5'- G GTG CAT GAC ACT GTF ACC TAT CCT CAG CG (SEQ ID NO: 10)(-YY) -3' (F = furanyl abasic site; YY = Yakima yellow tag). The complementary oligonucleotide had the sequence 5'- CG CTG AGG ATA GGT CAC AGT GTC ATG CAC C -3' (SEQ ID NO: 9).

Also provided was the 32-mer OGG1 oligonucleotide substrate containing 8-oxoguanine (Figure 2A) without the 3' Yakima yellow fluorescent tag, having the sequence 5'- CCG GTG CAT GAC ACT GTX ACC TAT CCT CAG CG -3' (SEQ ID NO: 11) (X = 8-oxoguanine).

***Robotic analysis of BER activities:*** All the assays were adapted to a robotic platform, in which liquid handling of the nicking reactions were performed automatically by a Freedom EVO 200 robot (Tecan, Mannedorf, GmBH) and Freedom EVOware software (Tecan, GmBH). Denatured radioactive DNA products were analyzed by electrophoresis on a 15 % polyacrylamide gel containing 8M urea, in 89 mM Tris-borate, 2.5 mM EDTA pH 8.0, at 1,500 V for 2 hours at 45-50 °C. The separation of radiolabeled DNA products was visualized and quantified using a Fuji BAS 2500 phosphorimager (Fuji, Valhalla, NY). Denatured fluorescent DNA products were analyzed by capillary gel electrophoresis, using the ABI3130XL genetic analyzer (Applied Biosystems, Foster City, CA), GeneMapper software (Applied Biosystems) and PeakAnalyzer software (Robiotec). Following, are the descriptions of the reaction conditions for each of the assays:
***MPG-Hx assay:*** The reaction mixture (20 µl) contained 50 mM PIPES (pH 6.7), 10 mM Tris (pH 7.1), 2 mM EDTA, 0.5 mM MgCl₂, 30 mM KCl, 1 µg/µl bovine γ-globulin, 0.1% polyvinyl alcohol (PVA), 7.5 nM substrate and 15 ng/µl protein extract. The reaction was carried out at 37 °C for 15 minutes, after which it was stopped by heat inactivation at 95°C for 2 minutes. The proteins were degraded by incubation with proteinase K (20 µg) for 30 minutes at 37 °C, after which they were treated with 100 mM NaOH for 30 minutes at 37 °C. One unit of MPG-Hx activity is defined herein as the amount required to cleave 1 fmol of DNA substrate in 1 hour at 37 °C, under the standard reaction conditions described herein. In the following, MPG-Hx is presented as specific activity, i.e., activity units/1 µg of total protein extract.
***MPG***-***eA assay:*** The reaction mixture (20 µl) contained 50 mM PIPES (pH 6.7), 10 mM Tris (pH 7.1), 2 mM EDTA, 0.5 mM MgCl₂, 30 mM KCl, 1 µg/µl bovine γ-globulin, 0.1% PVA, 0.5 nM substrate and 15 ng/µl protein extract. The reaction was carried out at 37 °C for 15 minutes, after which it was stopped by heat inactivation at 95°C for 2 minutes. The proteins were degraded by incubation with proteinase K (20 µg) for 30 minutes at 37 °C, after which they were treated with 100 mM NaOH for 30 minutes at 37 °C. One unit of MPG-eA activity is defined herein as the amount required to cleave 1 fmol of DNA substrate in 1 hour at 37 °C, under the standard reaction conditions described herein. In the following, MPG-eA is presented as specific activity, i.e., activity units/1 µg of total protein extract.
***OGG-F assay:*** The reaction mixture (10 µl) contained 50 mM Tris (pH 7.1), 1 mM EDTA, 115 mM KCl, 1 µg/µl bovine γ-globulin, 100 nM PolydA·polydT, 12.5 nM substrate and 0.2-0.5 µg /µl protein extract. The reaction was carried out at 37 °C for 30 minutes, after which it was stopped by heat inactivation at 95°C for 2 minutes. The reactions were treated with 100 mM NaOH for 30 minutes at 37 °C. One unit of OGG-F activity is defined herein as the amount required to cleave 1 fmol of DNA substrate in 1 hour at 37 °C, under the standard reaction conditions described herein. In the following, OGG-F is presented as specific activity, i.e., activity units/1 µg of total protein extract.
***MPG***-***Hx***-***F assay:*** The reaction mixture (20 µl) contained 50 mM MOPS (pH 6.8), 30 mM Tris (pH 7.1), 2 mM EDTA, 0.5 mM MgCl₂, 36 mM KCl, 1 µg/µl bovine γ-globulin, 0.3% PVA, 15 nM substrate and 45 ng/µl protein extract. The reaction was carried out at 37 °C for 15 minutes, after which it was stopped by heat inactivation at 95°C for 2 minutes. The reactions were treated with 100 mM NaOH for 30 minutes at 37 °C. One unit of MPG-Hx-F activity is defined herein as the amount required to cleave 1 fmol of DNA substrate in 1 hour at 37 °C, under the standard reaction conditions described herein. In the following, MPG-Hx-F is presented as specific activity, i.e., activity units/1 µg of total protein extract.
***APE1 assay:*** The reaction mixture (20 µl) contained 75 mM Tris (pH 7.8), 0.1 mM EDTA, 9 mM MgCl₂, 42.4 mM KCl, 0.25 µg/µl bovine γ-globulin, 0.25% PVA, 0.25 mM Spermidine; 0.05 mM Spermine; 35 nM substrate and 0.02 ng/µl protein extract. The reaction was carried out at 37 °C for 15 minutes, after which it was stopped by heat inactivation at 95°C for 2 minutes. The proteins were degraded by incubation with proteinase K (20 µg) for 30 minutes at 37 °C. One unit of APE activity is defined herein as the amount required to cleave 1 fmol of DNA substrate in 1 hour at 37 °C, under the standard reaction conditions described herein. In the following, APE is presented as specific activity, i.e., activity units/1 ng of total protein extract.
***APE1***-***F assay:*** The reaction mixture (20 µl) contained 75 mM Tris (pH 7.8), 0.1 mM EDTA, 9 mM MgCl₂, 42.4 mM KCl, 0.25 µg/µl bovine γ-globulin, 0.25% PVA, 0.25 mM Spermidine; 0.05 mM Spermine; 35 nM substrate and 0.015 ng/µl protein extract. The reaction was carried out at 37 °C for 15 minutes, after which it was stopped by heat inactivation at 95°C for 2 minutes. One unit of APE activity is defined herein as the amount required to cleave 1 fmol of DNA substrate in 1 hour at 37 °C, under the standard reaction conditions described herein. In the following, APE is presented as specific activity, i.e., activity units/1 ng of total protein extract.

### Statistical analysis:

The relative risk of lung cancer was estimated for MPG-Hx and MPG eA tests using conditional logistic regression models with smoking status as an adjusting variable.

Relative risks were estimated for each test as a continuous variable (assuming a linear relation with the log odds ratio), a binary variable using the median of the controls as the threshold, and categorized into three groups according to the tertiles of the controls. In the latter case a test for a linear trend in the log odds ratio was conducted using scores of 1, 2 and 3 for the three tertile groups. In some cases the relative risks were estimated per standard deviation of the range of enzyme activities of the reference (e.g. healthy) population.

The relative risk of lung cancer was estimated for pairwise combinations of MPG tests; OGG and MPG tests; APE and MPG tests and OGG and APE tests using conditional logistic regression models with smoking status as an adjusting variable. For the OGG and APE tests the odds ratio were calculated between a person at the 25% percentile versus a person at the 75% percentile of the distribution of assays values among the controls. For the MPG tests the odds ratio were calculated between a person at the 75% percentile versus a person at the 25% percentile of the distribution of assays values among the controls.

The relative risk of lung cancer was estimated for three-test combinations of MPG, APE and OGG tests using conditional logistic regression models with smoking status as an adjusting variable. For the OGG and APE tests the odds ratio were calculated between a person at the 25% percentile versus a person at the 75% percentile of the distribution of assays values among the controls. For the MPG tests the odds ratio were calculated between a person at the 75% percentile versus a person at the 25% percentile of the distribution of assays values among the controls.

The integrated DNA repair (OMA) score was obtained directly from the fit of the conditional logistic regression model including APE-F, OGG-F and MPG-Hx-F (with smoking status included in the model, see Table 8), and was defined, in this case, as 0.00425xAPE-F + 0.542xOGG-F - 0.0254xMPG-Hx-F. The odds ratio of lung cancer was estimated for the integrated DNA repair score using conditional logistic regression models adjusting for smoking status. Odds ratios were estimated for the continuous variable (assuming a linear relation with the log odds), the dichotomized variable, and categorized into three groups according to tertile. Because the weights for each component used in the integrated DNA repair score were chosen to optimize strength of association of the score with lung cancer for the observed data, a cross-validation procedure was applied for the dichotomized and three-category variables. The odds ratios unadjusted and adjusted by cross-validation are presented.

All the statistical analyses were performed using S-Plus program (TIBCO Software Inc.) and /or SAS software (version 9.2; SAS Institute Inc.).

### EXAMPLE I N-Methylguanine DNA glycosylase catalytic activity in peripheral blood cells

Base excision repair activity of N-Methylguanine DNA glycosylase (MPG) was assessed in protein extracts of peripheral blood mononuclear cells (PBMC) from blood samples of cancer patients and healthy controls, in order to uncover correlation between enzyme activity levels and incidence of disease.

### Results

***MPG Assays:*** MPG can initiate DNA repair by base excision (Figure 1) of a number of lesions in DNA, including hypoxanthine, alkylated bases such as 3-methyladenine and 7-methylguanine and secondary oxidative lesions such as 1,N6-ethenoadenine (Figure 2A-2E). Using a radioactive oligomer with a hypoxanthine (Hx) lesion, or a 1,N6 ethenoadenine (eA) lesion as substrate, conditions for accurate measurement of MPG activity were determined.

As can be seen in Figures 3A-3F, which shows gels illustrating the results of an MPG Hx assay (Figures 3C and 3E) and the quantitative expression of these results in a graph (Figures 3D and 3F), the assay was linear with time (Figures 3C and 3D) and protein concentration (Figures 3E and 3F). No cleavage was observed of the control oligonucleotide, which has an adenine (A) instead of Hx (Figures 3C and 3E). The assay was adapted to a robotic platform, in which liquid handling and nicking reactions were performed automatically in a Tecan robot robotic platform ([liquid handling of the nicking reactions were performed automatically by a Freedom EVO 200 robot and Freedom EVOware software (Tecan, GmBH)]. Analysis was done manually by separating the reaction products on a denaturing PAGE-urea gel, followed by drying the gels, and quantification by phosphorimaging. The assay was robust and reproducible with a coefficient of variance (CV) of about 10%.

Accurate assay conditions were established for substrate having an eA lesion as well. The assay was a nicking assay similar to the MPG-Hx assay, except that the substrate oligonucleotide contained 1, N6 ethenoadenine (eA) (Figure 5A), a site-specific exocyclic adduct of adenine. Activity of MPG from human PBMC extracts using the eA-lesioned substrate was found to be linear with time, over a range of greater than 2 hours (Figure 5D) and protein concentration, over a range of 0-30 ng/µl protein (Figure 5E). The absolute specific activity values for MPG-eA were about an order of magnitude lower than MPG-Hx, consistent with the reported higher activity of MPG on Hx-containing substrates compared to eA-containing substrates. The assay was reproducible with a CV of 8-9%.

### PBMC MPG activity is elevated in lung cancer

A case control study was undertaken to determine the association between lung cancer risk and MPG activity in protein extracts of PBMCs.

The case-control study was population based, with case patients and control subjects living in the North of Israel. PBMC were obtained from 100 lung cancer cases, and 100 control subjects matched for age (±1 years), sex and ethnicity. Table 1 below shows the demographic characteristics of lung cancer patients and controls. The mean average age of cancer patients and controls was 67, with 60% males and 40% females, 77% Jews and 23% non-Jews. The cancer patients group contained 38% current smokers, 37% former smokers and 25% life-long non-smokers. The control group contained 22% current smokers, 28% former smokers, and 50% life-long non-smokers. Blood specimens from patients were obtained before any treatment. PBMC were isolated within 24 hours, frozen in liquid nitrogen, and stored at -80°C, as described herein.

**Table 1. Demographic characteristics of case patients and control subjects**

| | **Cancer patients** | | | **Controls** | | | |
|---|---|---|---|---|---|---|---|
| **Study variable** | **Mean (95% CI)¹** | **N** | **%** | **Mean (95% CI)¹** | **N** | **%** | **P value²** |
| **Age** | 67.0 (65.1-68.8) | 100 | 100 | 67.0 (65.2-68.8) | 100 | 100 | 0.9877 |
| | | | | | | | |
| **Gender** | | | | | | | 1.00 |
| **Males** | | 60 | 60 | | 60 | 60 | |
| **Females** | | 40 | 40 | | 40 | 40 | |
| | | | | | | | |
| **Religion** | | | | | | | 0.87 |
| **Jews** | | 77 | 77 | | 77 | 77 | |
| **Non-Jews** | | 23 | 23 | | 23 | 23 | |
| | | | | | | | |
| **Smoking Status** | | | | | | | 0.001 |
| **Current** | | 37 | 38 | | 22 | 22 | |
| **Former** | | 36 | 37 | | 28 | 28 | |
| **Never** | | 24 | 25 | | 50 | 50 | |
| | | | | | | | |
| **Cancer Histology** | | | | | | | |
| **Adenocarcinoma** | | 46 | 46 | | | | |
| **SQCC** | | 30 | 30 | | | | |
| **BAC** | | 14 | 14 | | | | |
| **Other** | | 10 | 10 | | | | |

| | | | | | | | |
|---|---|---|---|---|---|---|---|
| ¹ 95% CI, 95% confidence interval. ² The results of two-sided Student's *t* tests between the patients and control subjects. | | | | | | | |

Fig. 4 shows the distributions of MPG enzymatic activity on the Hx substrate (MPG-Hx) in protein extracts prepared from PBMC obtained from 100 pairs of patients and controls. Surprisingly, the values of MPG-Hx activity in protein extracts from cancer patients were shifted to higher values than those of control subjects (Fig. 4), the converse of the results previously observed for activity of OGG-1, another DNA repair enzyme, when measured in PBMCs (Paz-Elizur et al., 2003, J Natl Can Inst;95:1312-19). Consistently, the average MPG-Hx specific activity was higher in lung cancer patients (174 units/µg protein, 95% CI 168-181) than in control subjects (161 units/µg protein, 95% CI 154-168; P=0.0032).

Conditional logistic regression analysis was performed for matched sets adjusted for smoking status to calculate the estimated relative risk associated with increased MPG-Hx activity. As can be seen in Table 2 below, individuals in the highest tertile of MPG-Hx activity had a increased risk of lung cancer compared with individuals in the lowest tertile (OR = 3.4, 95% CI 1.3-8.5; *P*=0.009). ). When the MPG-Hx activity was used as a continuous variable the adjusted odds ratio for lung cancer associated with 10 units increase in MPG-Hx activity was statistically significantly increased (OR = 1.18, 95% CI 1.05-1.33; P=0.006). Similarly the adjusted odds ratio for lung cancer associated with one standard deviation (SD) unit increase in MPG-Hx activity was significantly greater than 1 (OR=1.8; 95% CI=1.2 to 2.6; P=0.006). The adjusted odds ratio (OR) associated. For the subject with the highest MPG-Hx activity in this study (271 units/µg protein) this represents an increased estimated relative risk of 12.7-fold, when compared to the 10% percentile of healthy subjects.

**Table 2. Conditional logistic regression analysis of MPG-Hx enzymatic activity value in lung cancer patients and control subjects***

| **Variable** | **Number of patients (%)** | **Number of control subjects (%)** | **Adjusted† OR (95% CI)** |
|---|---|---|---|
| **MPG-Hx (per 10 U increase)‡** | 94 (100.0) | 94 (100.0) | 1.18 (1.05 to 1.33) |
| | | | P=0.006 |
| **MPG-Hx (per 1 SD increase)** 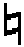 | 94 (100.0) | 94 (100.0) | 1.8 (1.2 to 2.6) |
| | | | P=0.006 |
| **MPG-Hx (by median)§** | 26 (27.7) | 47 (50.0) | 1.0 (referent) |
| **≤159 U** | 68 (72.3) | 47 (50.0) | 3.3 (1.5 to 7.2) *P*=0.002 |
| **>159 U** | | | |
| **MPG-Hx (by tertiles)#** | 16 (17.0) | 32 (34.0) | 1.0 (referent) |
| **≤146 U** | 31 (33.0) | 30 (31.9) | 1.7 (0.8 to 4.0) *P*=0.19 |
| **146-171 U** | 47 (50.0) | 32 (34.0) | 3.4 (1.3 to 8.5) *P*=0.009 |
| **>171 U** | | | Trend test¶ P=0.009 |

| | | | |
|---|---|---|---|
| *OR = odds ratio; CI = confidence interval. †Conditional logistic regression for matched sets adjusted for age and smoking status (smoker, nonsmoker). ‡MPG-Hx activity was measured as described in the text and was fitted in the conditional logistic regression model as a continuous variable. The odds ratio for smoking, obtained with this model, was: ex-smoker v never smoker: 3.6 (95% CI= 1.4 to 8.9); current smoker v never smoker: 4.0 (1.7 to 9.5). 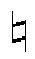 MPG-Hx activity expressed in standard deviation (SD) units to allow meaningful comparison. 34 U represent 1 SD in the control group. §Median of the control subjects' values. #Tertiles of the control subjects' values. The lower tertile was chosen as the referent. ¶Adjusted for the same variable as described above. | | | |

Figure 6 shows the distributions of MPG enzyme activity on the eA substrate (MPG-eA) in the protein extracts prepared from PBMC obtained from the same 100 pairs of lung cancer patients and controls. As with the MPG-Hx assay the values of MPG-eA activity in protein extracts from patients were shifted to higher values than controls. Consistently, the average specific activity was higher in patients (15.8 units/µg protein; 95% CI 15.3-16.3), than in controls (15.1 units/µg protein, 95%CI 14.6-15.6; P=0.0051 paired t-test).

Conditional logistic regression analysis was performed for matched sets adjusted for smoking status to calculate the estimated relative risk associated with increased MPG-eA activity. Individuals in the highest tertile of MPG-eA activity had an increased risk of lung cancer compared with individuals in the lowest tertile (OR =2.2, 95% CI = 0.9-4.9; P=0.07) (Table 3). When the MPG-eA activity was used as a continuous variable the adjusted odds ratio for lung cancer associated with 1 unit increase in MPG-eA activity was statistically significantly increased (OR = 1.22, 95% CI = 1.04-1.42; P=0.013) (Table 3). Similarly the adjusted odds ratio for lung cancer associated with one standard deviation (SD) unit increase in MPG-eA activity was significantly greater than 1 (OR=1.6; 95% CI=1.1 to 2.4; P=0.013). For the individual with the highest MPG-εA activity in this study (21.9 units/µg protein) this represents an increased estimated relative risk of 7.6-fold, when compared with that of the 10% percentile of healthy subjects.

**Table 3. Conditional logistic regression analysis of MPG-eA enzymatic activity value in lung cancer patients and control subjects***

| **Variable** | **Number of patients (%)** | **Number of control subjects (%)** | **Adjusted† OR (95% CI)** |
|---|---|---|---|
| **MPG-eA (per 1 U increase)‡** 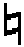 | 96 (100.0) | 96 (100.0) | 1.22 (1.04 to 1.42) *P*=0.013 |
| **MPG-eA (per 1 SD increase)** | 96 (100.0) | 96 (100.0) | 1.6 (1.1 to 2.4) *P*=0.013 |
| **MPG-eA (by median)§** | 35 (36.5) | 48 (50.0) | 1.0 (referent) |
| **≤15.1 U** | 61 (63.5) | 48 (50.0) | 1.9 (0.9 to 3.9) *P*=0.08 |
| **>15.1 U** | | | |
| **MPG-eA (by tertiles)#** | 24 (25.0) | 32 (33.3) | 1.0 (referent) |
| **≤14.1 U** | 27 (28.1) | 32 (33.3) | 1.0 (0.5 to 2.3) *P*=0.95 |
| **14.1-16.1 U** | 45 (46.9) | 32 (33.3) | 2.2 (0.9 to 4.9) *P*=0.07 |
| **>16.1 U** | | | Trend test¶ *P*=0.07 |

| | | | |
|---|---|---|---|
| *OR = odds ratio; CI = confidence interval. †Conditional logistic regression for matched sets adjusted for age and smoking status (smoker, nonsmoker). ‡MPG-εA activity was measured as described in the text and was fitted in the conditional logistic regression model as a continuous variable. The odds ratio for smoking, obtained with this model, was: ex-smoker v never smoker: 3.5 (95% CI= 1.4 to 8.4); current smoker v never smoker: 3.6 (1.5 to 8.2). 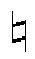 MPG-eA activity expressed in standard deviation (SD) units to allow meaningful comparison. 2.48 U represent 1 SD in the control group. §Median of the control subjects' values. #Tertiles of the control subjects' values. The lower tertile was chosen as the referent. ¶Adjusted for the same variable as described above. | | | |

Conditional logistic regression of disease status on a pairwise combination of the MPG-Hx and MPG-eA assays adjusted for smoking status was calculated, in order to examine whether a combination of results from the MPG-Hx and MPG-eA assays would provide a stronger association with lung cancer. The assays were not found to enhance each other (Table 4), which is also consistent with the observation that they are highly correlated to each other (correlation of 0.76).

**Table 4. Conditional logistic regression of disease status on a pairwise combination of MPG-Hx and MPG-eA DNA repair assays adjusted for smoking status**

| **Assay** | **OR (95% CI)** | **P-value** | **Joint OR (95% CI)** | **Overall P-value** | **Significantly better?** |
|---|---|---|---|---|---|
| | | | | | |
| MPG-Hx | 1.5 (0.8,2.9) | 0.24 | 2.1 (1.2,3.5) | 0.010 | **NO** |
| MPG-eA | 1.4 (0.6, 3.1) | 0.44 | | | |

The odds ratio is calculated between a person at the 75% percentile versus a person at the 25% percentile of the distribution of assay values among the controls.

Thus, assay of MPG activity in PBMCs, using either Hx or eA substrates, provides a practically identical correlation with risk for lung cancer. Taken together, these results show that elevated activity of MPG is an accurate and easily assessed risk factor for lung cancer.

### EXAMPLE II: Fluorescence-based DNA base excision repair enzyme assay

Fluorescence-labeled substrates can be more convenient than radioactive-labeled substrates. However, a fluorescent label, which is a foreign moiety in the actual DNA structure, might possibly be identified as 'DNA damage' by the repair enzymes, therefore affecting catalytic activity in an undesired, artifactual manner.

Decreased catalytic activity of 8-oxoguanine DNA glycosylase (OGG), measured in PBMCs, has been shown to correlate with higher risk for lung cancer (see Paz-Elizur et al. J Natl. Canc. Inst. 2003;95:1312-19). The results shown herein clearly indicate that high MPG activity, measured in PBMCs, is also an accurate risk biomarker for lung cancer. In order to examine whether fluorescence-tagged substrates can be used to assay these and other DNA repair enzyme activities in place of the radioactive substrates, fluorescent-labeled DNA oligomer substrates for OGG and MPG catalytic activity were synthesized and tested with PBMC extract.

### Results

### Fluorescent-labeled DNA substrates are accurately excised by DNA repair enzymes:

Figure 7 shows the distribution of OGG-F enzyme activity in PBMC protein extracts from the same 100 pairs of cancer patients and controls as described in Example I. As with the OGG radioactivity-based assay previously described (see Elizur-Paz, 2008), the values of OGG-F activity in protein extracts from patients were shifted to lower values than controls (Figure 7). Consistently, the average specific activity was lower in cases (6.3 units/µg protein, 95% CI 6.1-6.6), when compare with that of controls (6.8 units/µg protein; 95% CI 6.6-7.0; *P*=0.0031 paired t-test).

Conditional logistic regression of disease status on the OGG-F DNA repair assay adjusted for smoking status was performed with 96 pairs of case patients and control subjects. The odds ratio values were calculated between a person at the 25% percentile versus and a person at the 75% percentile of the distribution of assay values among the controls, yielding a value of OR=1.6 (95% CI 1.1-2.3), *P*=0.011. Thus, low OGG activity in PBMCs, when assayed with a fluorescent-labeled substrate is an accurate risk biomarker for lung cancer, just as when assayed with a radio-labeled substrate.

### EXAMPLE III: Combining two DNA repair enzyme activities can provide synergically enhanced risk assessment

Pairwise combinations of the OGG-F, MPG-Hx and MPG-eA DNA repair enzyme assays of the same specimens were examined, using conditional logistic regression adjusted for smoking status. The results presented in Table 5 show that the combination of OGG-F and MPG-Hx gave an OR of 3.7 (95% CI 1.8-7.7), with P=0.0002, significantly better than each assay alone. Similarly, the combination of OGG-F and MPG-eA gave a joint OR of 6.7 (95% CI 2.4-16.7), P=0.00002, significantly better than each assay alone (Table 5).

**Table 5. Results of conditional logistic regression of disease status on a pairwise combination of OGG and MPG DNA repair assays adjusted for smoking status**

| **Assay** | **OR (95% CI)** | **P-value** | **Joint OR (95% CI)** | **Overall P-value** | **Significantly better than individual OR?** |
|---|---|---|---|---|---|
| **OGG-F** | 1.8 (1.2, 2.6) | 0.007 | 3.7 (1.8, 7.7) | 0.0002 | **YES** |
| **MPG-Hx** | 2.1 (1.3,3.4) | 0.002 | | | |
| **OGG-F** | 2.2 (1.4,3.4) | 0.0008 | 6.7 (2.4, 16.7) | 0.00002 | **YES** |
| **MPG-eA** | 2.9 (1.6,5.6) | 0.0009 | | | |
| **OGG-F** | 1.9 (1.2,2.8) | 0.003 | 3.6 (1.5, 8.3) | 0.002 | **YES** |
| **MPG-Hx-F** | 1.9 (1.1, 3.5) | 0.033 | | | |

The odds ratio for OGG was calculated between a person at the 25% percentile versus a reference person at the 75% percentile of the distribution of assay values among the controls. The odds ratio for MPG was calculated between a person at the 75% percentile versus a reference person at the 25% percentile of the distribution of assay values among the controls.

Included in Table 5 are results from assays using a fluorescent MPG-Hx substrate, prepared and assayed as described in the Materials and Methods section of the Examples section below. The correlation of the MPG-Hx-F assay to the ³²P-based MPG-Hx assay was 0.87, suggesting that the two perform similarly. Conditional logistic regression of the OGG-F and MPG-Hx-F pair yielded a joint OR of 3.6 (95% CI 1.5-8.3), *P*=0.002, significantly better than each assay alone (Table 5). These results are very similar to the results obtained with the [OGG-F, MPG-Hx] pair, and show that when paired, the OGG and MPG assays are a significantly more powerful risk biomarker for lung cancer than either biomarker assayed alone (Table 5).

### EXAMPLE IV Apurinic/apyrimidinic endonuclease 1 catalytic activity in peripheral blood cells

Abasic DNA incision repair activity of apurinic/apyrimidinic endonuclease 1 (APE-1, APE) is responsible for nicking of the abasic DNA site formed following base excision by DNA glycosylases such as OGG and MPG (Figure 1). APE 1 catalytic activity was assessed in protein extracts of peripheral blood mononuclear cells (PBMC) from blood samples of cancer patients and healthy controls, in order to uncover correlation between enzyme activity levels and incidence of disease.

Assay conditions, preparation of samples and statistical analyses were performed as detailed for OGG and MPG in the Materials and Methods section hereinbelow.

### Results

DNA oligomers bearing the synthetic abasic site (furanyl abasic site; Figure 2E left and 8A) have been previously shown to be a good substrate for APE1 activity. The assay was optimized to yield highly reproducible results, yielding a CV of about 15%.

Figures 8A- 8E show kinetics and protein titration of APE1 activity in extracts from human PBMC. The nicking activity of APE1 at the abasic site of the substrate was found to be linear over at least the range of 0.005 to 0.020 ng/µl protein per sample, and 0-60 minutes incubation time (FIGs. 8D and 8E). Somewhat surprisingly, human PBMC contain a very high enzymatic activity of APE1, about 100,000-fold higher than OGG or MPG-eA activities.

### APE-1 activity in PBMC is a reliable predictor of risk of lung cancer

Figure 9 shows the distributions of APE1 enzyme activity in the PBMC protein extracts from the same 100 pairs of patients and controls which were assayed previously for OGG and MPG. APE1 activity in protein extracts from cases was shifted to lower values than controls. Consistently, the average specific activity was lower in cases (692 units/ng protein; 95% CI 656-728), than in controls (793 units/ng protein, 95% CI 751-835, P<0.0001).

**Table 6. Conditional logistic regression analysis of APE1 enzymatic activity value in lung cancer patients and control subjects***

| **Variable** 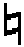 | **Number of patients (%)** | **Number of control subjects (%)** | **Adjusted† OR (95% CI)** |
|---|---|---|---|
| **APE (per 100 U decrease)‡** | 96(100.0) | 96(100.0) | 1.4 (1.1 to 1.7) |
| | | | *P*=*0.002* |
| **APE (per 1 SD decrease)** | 96(100.0) | 96 (100.0) | 2.0(1.3 to 3.1) |
| | | | *P*=*0.002* |
| **APE (by median)§** | | | |
| **>785 U** | 27(28.1) | 48(50.0) | 1.0 (referent) |
| **≤785 U** | 69(71.9) | 48(50.0) | 2.6 (1.2 to 5.4) *P*=0.01 |
| **APE (by tertiles)#** | | | |
| **>847 U** | 19(19.8) | 32 (33.3) | 1.0 (referent) |
| **718-847 U** | 17(17.7) | 32 (33.3) | 0.9 (0.3 to 2.2) *P*=0.77 |
| **≤718 U** | 60 (62.5) | 32 (33.3) | 3.3 (1.4 to 8.1) *P*=0.008 |
| | | | Trend test¶ P=0.004 |

| | | | |
|---|---|---|---|
| *OR = odds ratio; CI = confidence interval. †Conditional logistic regression for matched sets adjusted for smoking status (smoker, ex-smoker, never smoker). ‡APE activity was measured as described in the "Materials and Methods" section and was fitted in the conditional logistic regression model as a continuous variable. The odds ratio for smoking, obtained with this model, was: ex-smoker v never smoker: 3.4 (95% CI= 1.4 to 8.2); current smoker v never smoker: 2.6 (1.1 to 6.1). 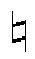 APE activity expressed in standard deviation (SD) units to allow meaningful comparison. 211 U represent 1 SD in the control group. §Median of the control subjects' values. #Tertiles of the control subjects' values. The upper tertile was chosen as the referent. ¶Adjusted for the same variable as described above. | | | |

### Materials and Methods

Conditional logistic regression analysis was performed for matched sets adjusted for smoking status to calculate the estimated relative risk associated with decreased APE1 activity. As can be seen in Table 6 above, individuals in the lowest tertile of APE1 activity had a increased risk of lung cancer compared with individuals in the higher tertile (OR = 3.3, 95% CI 1.4-8.1; *P*=0.008). When the APE1 activity was used as a continuous variable the adjusted odds ratio for lung cancer associated with 100 units decrease in APE1 activity was statistically significantly increased (OR = 1.4, 95% CI 1.1-1.7; P=0.002). Similarly the adjusted odds ratio for lung cancer associated with one standard deviation (SD) unit decrease in APE1 activity was significantly greater than 1 (OR=2.0; 95% CI=1.3 to 3.1; *P*=0.002). For the subject with the lowest APE1 activity in this study (348 units/ng protein) this represents a decreased estimated relative risk of 9.5-fold relative to the 90% percentile of healthy subjects. These results indicate that reduced APE1 activity is a reliable biomarker for the risk of lung cancer. Thus, we have identified 3 risk biomarkers for lung cancer: reduced activity of OGG, reduced activity of APE, and increased activity of MPG.

### EXAMPLE V: Combining base excision and abasic incision DNA repair enzyme activity values and cancer risk assessment

Combinations of the OGG-F, MPG-Hx and MPG-eA base excision and APE1 abasic DNA incision DNA repair enzyme assays of the same specimens were examined, using conditional logistic regression adjusted for smoking status, in order to uncover combinations of enzyme activity that can be assayed together, and that provide advantageous sensitivity (higher odds ratio) than that of the individual enzyme assays.

Assay conditions, preparation of samples and statistical analyses were performed as detailed for OGG, MPG and APE1 in Examples I-IV.

### Results

***MPG-APE1 combination:*** Combination of MPG and APE1 were examined using the results of the DNA repair assays, by conditional logistic regression adjusted for smoking status, for pairwise combinations of APE and MPG. As can be seen in Table 7, for the [APE, MPG-Hx] pair the paired OR was 10 (95% CI 3.2-33), with P<0.00001, significantly better than that of each assay alone (Table 6). A similar analysis was performed for the [APE, MPG-eA] pair, yielding a paired OR of 5.6 (95% CI 1.9-16.7), with P=0.0002, significantly better than that of each assay alone (Table 6).

**Table 7. Results of conditional logistic regression of disease status on pairwise combinations of (APE, MPG) and (OGG, APE) DNA repair assays, adjusted for smoking status**

| **Assay** | **OR (95% CI)** | **P-value** | **Joint OR (95% CI)** | **Overall P-value** | **Significantly better?** |
|---|---|---|---|---|---|
| **APE-MPG pairs** | | | | | |
| **APE** | 3.7 (1.7,7.1) | 0.00004 | 10(3.2,33) | P<0.00001 | **YES** |
| **MPG**-**Hx** | 2.7 (1.5,5.1) | 0.002 | | | |
| | | | | | |
| **APE** | 2.9 (1.6,5.3) | 0.0003 | 5.6 (1.9, 16.7) | P=0.0002 | **YES** |
| **MPG**-**eA** | 1.9 (1.0,3.4) | 0.04 | | | |
| | | | | | |
| **APE**-**F** | 4.2 (2.0,9.1) | 0.0002 | 9.1 (2.5, 33) | P=0.0001 | **YES** |
| **MPG**-**Hx**-**F** | 2.2 (1.2,4.1) | 0.015 | | | |

| **OGG**-**APE pairs** | | | | | |
|---|---|---|---|---|---|
| **OGG-F** | 1.8 (1.1, 3.1) | 0.029 | 2.8 (1.5, 5.0) | P=0.0002 | **NO** |
| **APE** | 1.5 (0.9, 2.6) | 0.14 | | | |
| | | | | | |
| **OGG-F** | 1.9 (1.1, 3.2) | 0.026 | 2.8 (1.5, 5.9) | P=0.0002 | **NO** |
| **APE-F** | 1.5 (0.8,2.7) | 0.17 | | | |

The odds ratios for OGG and APE were calculated between a person at the 25% percentile versus a reference person at the 75% percentile of the distribution of assay values among the controls. The odds ratio for MPG was calculated between a person at the 75% percentile versus a reference person at the 25% percentile of the distribution of assay values among the controls.

Included in Table 7 are results from assays using a fluorescent APE1 substrate (APE-F), prepared and assayed as described herein. Conditional logistic regression of the APE-F and MPG-Hx-F pair yielded a paired OR of 9.1 (95% CI 2.5-33), *P*<0.00001, significantly better than each assay alone (Table 7). These results are very similar to the results obtained with the [APE, MPG-Hx] pair, and show that the combined pair of APE1 and MPG assays is a more powerful risk biomarker for lung cancer than each biomarker alone.

***OGG-F APE1 Combination:*** The combination of OGG-F and APE1 risk biomarkers was also assessed for advantage over individual efficacy. Conditional logistic regression of the APE and OGG-F pair yielded a paired OR of 2.8 (95% CI 1.5-5.0), *P*=0.0002, which was clearly not significantly better than the OR of each assay alone (Table 7).

A similar result was obtained for the combination of the OGG-F and APE-F fluorescent biomarkers. Conditional logistic regression of the APE-F and OGG-F pair yielded a joint OR of 2.8 (95% CI 1.5-5.9), P=0.0002. This was not significantly better than each assay alone (Table 6). Thus, unlike the [APE, MPG] combination, the [APE, OGG] combination is not significantly better than the APE and OGG assays alone.

***MPG-APE1-OGG Combination:*** The combination of the results using three DNA repair enzymes, OGG, MPG and APE, was assessed, to see whether it might provide a better risk estimate than pairs of biomarkers, or each risk biomarker alone. This was done using the results of the DNA repair assays, by conditional logistic regression adjusted for smoking status, for triplet combinations of OGG, MPG and APE biomarkers. As can be seen in Table 8, the combined OR values obtained for the various assay combinations were between 12.1 to 24.8, each significantly better than single assays or two-assay combinations. It will be noted that, for example, the panel of the 3 fluorescence-based assays (OGG-F, MPG-Hx-F, APE-F) yielded a joint OR=24.8, indicating that it is an extremely powerful risk biomarker for lung cancer, when compared to single assays or two-assay combinations.

**Table 8. Results of conditional logistic regression of disease status on three-test combination of OGG-MPG-APE DNA repair assays adjusted for smoking status**

| **Assay** | **OR (95% CI)** | **P-value** | **Joint OR (95% CI)** | **Overall P-value** | **Significantly better?** |
|---|---|---|---|---|---|
| **OGG-F** | 2.2 (1.2, 4.2) | 0.013 | 18.3 | P<0.0001 | **YES** |
| **APE** | 2.5 (1.3, 5.0) | 0.007 | | | |
| **MPG-Hx** | 3.0 (1.6, 5.8) | <0.001 | | | |
| **OGG-F** | 2.3 (1.2, 4.3) | 0.008 | 12.1 | P<0.0001 | **YES** |
| **APE** | 2.1 (1.1, 3.8) | 0.02 | | | |
| **MPG-eA** | 2.4 (1.2, 4.6) | 0.011 | | | |
| **OGG-F** | 2.4 (1.3, 4.5) | 0.006 | 24.8 | P<0.0001 | **YES** |
| **APE-F** | 3.1 (1.4, 7.1) | 0.005 | | | |
| **MPG-Hx-F** | 2.9 (1.4, 5.6) | 0.004 | | | |
| **OGG-F** | 2.4 (1.3, 4.3) | 0.006 | 14.4 | P<0.0001 | **YES** |
| **APE-F** | 2.4 (1.2, 5.0) | 0.014 | | | |
| **MPG**-**eA** | 2.6 (1.3, 5.2) | 0.007 | | | |

The odds ratios for OGG and APE were calculated between a person at the 25% percentile versus a reference person at the 75% percentile of the distribution of assay values among the controls. The odds ratio for MPG was calculated between a person at the 75% percentile versus a reference person at the 25% percentile of the distribution of assay values among the controls.

Taken together, these results show that catalytic activity of both DNA repair enzymes MGP and APE1, although having different roles in DNA repair, can be an accurate predictor of lung cancer risk. Surprisingly, whereas low relative OGG and low APE1 values have been shown to indicate increased risk of lung cancer, increased risk of lung cancer correlates with elevated levels of MPG activity.

Yet further, combining results of activity levels of OGG, MPG and APE1 indicated that some, but not all combinations provide statistically powerful correlations with risk for lung cancer, the most powerful being a panel of all three repair enzymes, measured with fluorescent-labeled DNA oligonucleotide substrates having site-specific lesions.

### EXAMPLE VI- Combined Score for OGG, MPG and APE1 (OMA) is strongly associated with lung cancer risk

When measuring the three DNA repair activities in an individual, it is useful to form an integrated DNA repair score, which combines the three DNA repair measures with the appropriate weights. Such a score takes into accounts also individuals in whom only one or two of the DNA repair tests are sub-optimal, and is therefore better applicable to measure estimated relative risk. This integrated DNA repair score (OMA) was calculated for each study participant as described under Statistical Analysis Methods. FIG. 10 shows the distributions of the integrated DNA repair score (OMA) score among cases and controls. The distribution of integrated DNA repair score (OMA) scores among the cases (patients) is clearly shifted to lower DNA repair score (OMA) values, compared to the healthy controls DNA repair score (OMA) range among controls was 1.40-10.42 units, whereas among cases it was 0.53-7.01). The mean integrated DNA repair score (OMA) score among cases was 2.8 (95%CI 2.6 to 3.0), significantly lower than the mean (3.6 (95%CI 3.4 to 3.8)) among controls (*P*<0.0001) (Table 9). When further analyzed for specific cancers, it was found that the integrated DNA repair score (OMA) DNA repair score in adenocarcinoma cases was 2.9 (95%CI 2.7 to 3.2), higher than the 2.5 observed in squamous cell carcinoma (SQCC) cases (95%CI 2.2 to 2.7), *P*=0.04. There was no appreciable difference in the integrated DNA repair score (OMA) score between males and females, and between subjects >65 and ≤65 years old. In addition, there was no appreciable difference between never, former and current smokers, and no interaction between the integrated DNA repair score (OMA) score and smoking status.

**Table 9. Distribution of selected characteristics and integrated DNA repair (OMA) score in lung cancer patients and control subjects***

| Variable | Control participants (n=100) | | Case participants (n=100) | | *P*† |
|---|---|---|---|---|---|
| | | | | | |
| | | OMA score mean | | OMA score mean | |
| | No. | (95% CI) | No. | (95% CI) | |
| All^{∼} | 99 | 3.6 (3.4, 3.8) | 99 | 2.8 (2.6, 3.0) | <0.0001 |
| | | | | | |
| SQCC | | | 30 | 2.5 (2.2, 2.7) | |
| Adenocarcinoma | | | 45 | 2.9 (2.7, 3.2) | |
| | | | | §*P*=0.04 | #*P*=0.35 |
| Age, y | | | | | |
| ≤65 | 40 | 3.6 (3.3, 3.9) | 40 | 2.8 (2.5, 3.1) | |
| >65 | 59 | 3.6 (3.2, 3.9) | 59 | 2.8 (2.5, 3.1) | |
| | | | | | #*P*=0.91 |
| | ^*P*= 0.67 | | | | |
| Sex | | | | | |
| Male | 59 | 3.5 (3.3, 3.8) | 59 | 2.7 (2.5, 3.0) | |
| Female | 40 | 3.7 (3.3, 4.2) | 40 | 2.9 (2.5, 3.2) | |
| | | | | | #P=0.62 |
| | ^P=0.33 | | | | |
| Smoking status | | | | | |
| Never smoked | 50 | 3.7 (3.4, 4.1) | 24 | 2.8 (2.4, 3.2) | |
| Past smoker | 27 | 3.6 (3.2, 4.1) | 36 | 2.7 (2.4, 3.1) | |
| Current smoker | 22 | 3.3 (2.9, 3.7) | 36 | 2.8 (2.5, 3.1) | |
| | | | | | #P=0.52 |
| | ^P=0.39 | | | | |

| | | | | | |
|---|---|---|---|---|---|
| * Combined OMA score was defined as: 0.00425xAPE + 0.5419×OGG - 0.02541×MPG. One participant did not have known test values. This participant and the matched control were excluded from the analysis. Of the 100 lung cancer cases, 30 had squamous cell carcinoma (SQCC), 46 had adenocarcinoma, 14 BAC, 4 adenosquamous carcinoma, 4 adenoBAC; 1 small cell carcinoma, and 1 unknown histology. † Analysis of covariance comparing cases with controls, with matched pair and smoking status as a covariate. For smoking status subgroups, the covariates were age (continuous) and gender. § Analysis of covariance comparing subsets within cases or controls, with smoking status, age (continuous) or gender as covariates, as appropriate. ^Analysis of covariance comparing subsets stratified by cases and controls, with smoking status, age (continuous) or gender as covariates, as appropriate. # Test for interaction between case-control status and the variable of interest. | | | | | |

The association between the integrated DNA repair (OMA) score and the probability of having the disease, adjusted for smoking status, was calculated using conditional logistic regression. As can be seen in Table 10, low integrated DNA repair (OMA) score was associated with increased lung cancer risk, as indicated by the statistically significant odds ratios obtained using three logistic regression models.

When the integrated DNA repair (OMA) score was used as a continuous variable the adjusted odds ratio for lung cancer associated with one standard deviation (SD) unit decrease in integrated DNA repair (OMA) score was significantly greater than 1 (OR=3.2; 95% CI=1.7 to 5.7; *P*<0.001) (Table 10). When the integrated DNA repair (OMA) scores were dichotomized according to the median of the control subjects, the adjusted odds ratio for lung cancer associated with lower OMA score was 3.8 (95%CI =1.7 to 8.5; *P*=0.001). When integrated DNA repair (OMA) scores were divided into tertiles using the control subjects' values, the adjusted odds ratio for the lowest tertile versus the highest was 9.7 (95% CI=3.1 to 29.8; *P*<0.001). The odds ratio for the middle tertile was 3.3 (95% CI 1.1 to 9.5; *P*=0.03) (Table 10).

When calculating the weights for each component of the integrated DNA repair (OMA) score (i.e., the regression coefficients from conditional logistic regression with the three DNA repair assay values as well as smoking as explanatory variables), the weights are estimated so as to give the best discrimination between cases and controls for the available data. Cross-validation of the odds ratios was employed to obtain estimated odds ratios reflecting the likely performance of the score in a hypothetical new dataset. When calculated using cross validation, the adjusted odds ratio for lung cancer associated with low integrated DNA repair (OMA) score in the median model was 3.0 (95% CI 1.4 to 6.4) and in the tertiles model 5.6 (2.1 to 15.1), lower than in the original analysis, but still a very strong (Table 10).

**Table 10. Conditional logistic regression analysis of combined score based on OGG, APE and MPG values in lung cancer patients and control subjects***

| Variable | No. of case patients (%) | No. of control subjects (%) | Adjusted† OR (95% CI) |
|---|---|---|---|
| Score (per 1.18U)‡ | 96 (100.0) | 96 (100.0) | 3.2 (1.7 to 5.7) *P*<0.001 |
| Score (by median) § | | | |
| >3.51 | 21 (21.9) | 47 (49.0) | (referent) |
| ≤3.51 | 75 (78.1) | 49 (51.0) | 3.8 (1.7 to 8.5) *P*=0.001 |
| **Cross-validation** | | | |
| Above median | 22 (22.9) | 45 (46.9) | (referent) |
| Below median | 74 (77.1) | 51 (53.1) | 3.0 (1.4 to 6.4) |
| Score (by tertiles)# | | | |
| >3.98 | 8 (8.3) | 31 (32.3) | (referent) |
| 3.15-3.98 | 23 (24.0) | 32 (33.3) | 3.3 (1.1 to 9.5) *P*=0.03 |
| ≤3.14 | 65 (67.7) | 33 (34.4) | 9.7 (3.1 to 29.8) *P*<0.001 Trend test *P*<0.001 |
| **Cross validation** | | | |
| Highest tertile | 12(12.5) | 30(31.2) | (referent) |
| Middle tertitle | 19(19.8) | 33 (34.4) | 1.5 (0.6 to 3.7) |
| Lowest tertile | 65 (67.7) | 33 (34.4) | 5.6 (2.1 to 15.1) |

| | | | |
|---|---|---|---|
| * OR = odds ratio; CI = confidence interval. † Conditional logistic regression for matched sets adjusted for smoking status (smoker, ex-smoker, never smoker). ‡ Score was defined as 0.00425×APE-F + 0.542×OGG-F - 0.0254×MPG-Hx-F and was fitted in the conditional logistic regression model as a continuous variable. The value 1.18U is one standard deviation in the control group. The odds ratio for smoking, obtained with this model, was: ex-smoker v never smoker: 2.6 (95% CI= 1.0 to 6.3); current smoker v never smoker: 3.0 (1.1 to 8.2). § Median of the control subjects' values. # Tertiles of the control subjects' values. The upper tertile was chosen as the referent. | | | |

## Claims

1. A method of determining a risk of a human subject to develop lung cancer, the method comprising determining a level of catalytic activity of N-methylpurine DNA glycosylase (MPG) in a biological sample of the subject, and,
according to said level, determining the risk of the subject to develop lung cancer, wherein a level of said catalytic activity of MPG above a first predetermined value is indicative of an increased risk of said subject to develop lung cancer.

2. The method of claim 1, further comprising
(a) determining a level of catalytic activity of
(i) apurinic/apyrimidinic endonuclease 1 (APE1) or
(ii) 8-oxoguanine DNA glycosylase (OGG1) or
(iii) both apurinic/apyrimidinic endonuclease 1 (APE1) and 8-oxoguanine DNA glycosylase (OGG1)
in said biological sample, and;
according to said level, determining the risk of the subject to develop lung cancer, wherein a level of said catalytic activity of MPG above a first predetermined value and either a level of said catalytic activity of APE1 below a second predetermined value or a level of said catalytic activity of OGG1 below a third predetermined value or both a level of said catalytic activity of APE1 below said second predetermined value and a level of said catalytic activity of OGG1 below said third predetermined value
is indicative of an increased risk of said subject to develop lung cancer.

3. The method of claim 1, further comprising:
determining the level of catalytic activity of at least one of apurinic/apyrimidinic endonuclease 1 (APE1) and 8-oxoguanine DNA glycosylase (OGG1) in said biological sample of the subject;
(b) determining an integrated DNA repair score for said subject from said level of MPG and at least one of OGG1 and APE1; and
(c) determining the risk of the subject to develop lung cancer, wherein an integrated DNA repair score below a predetermined value is indicative of an increased risk of said subject to develop lung cancer.

4. The method of claim 2 or 3, wherein said determining said catalytic activity in (a) comprises determining a level of catalytic activity of N-methylpurine DNA glycosylase (MPG) and of apurinic/apyrimidinic endonuclease 1 (APE1) in said biological sample of the subject.

5. The method of claim 2 or 3, wherein said determining said catalytic activity in (a) comprises determining a level of catalytic activity of N-methylpurine DNA glycosylase (MPG) and 8-oxoguanine DNA glycosylase (OGG1) in said biological sample of the subject..

6. The method of claim 2 or 3, wherein said determining said catalytic activity in (a) comprises determining a level of catalytic activity of N-methylpurine DNA glycosylase (MPG), of apurinic/apyrimidinic endonuclease 1 (APE1) and of 8-oxoguanine DNA glycosylase (OGG1) in said biological sample of the subject.

7. The method of any one of claims 1-6, wherein said risk or risk level is expressed as an odds ratio (OR) as compared to the risk of developing lung cancer of that of a reference population of normal, apparently healthy individuals matched to said subject or subjects for at least one parameter selected from the group consisting of gender, age, ethnicity and smoking status.

8. The method of claim 7, wherein the odds ratio for MPG catalytic activity, when determined by the MPG-Hx assay, is 1.18 for each 10 units of catalytic activity or 1.8 for each 1 SD above said predetermined value.

9. The method of claim 7, wherein the combined odds ratio for MPG and APE1 catalytic activities, relative to that of said reference population, is at least 5, wherein the odds ratio for APE1 is determined by comparing APE1 catalytic activity at the 25^{th} percentile with those of the 75th percentile of control values and the odds ratio for MPG is determined by comparing MPG catalytic activity at the 75th percentile with those of the 25th percentile of control values.

10. The method of claim 7, wherein the odds ratio is calculated from an integrated DNA repair score for combined MPG, OGG1 and APE1 catalytic activity, wherein when said integrated DNA repair score is below the median of a reference population, said odds ratio is at least 3.0.

11. The method of any one of claims 1-10, wherein determining said MPG catalytic activity is effected using a double stranded oligonucleotide substrate having a hypoxanthine lesion (Hx) or using an oligonucleotide substrate having an N6-ethenoadenine lesion (eA), wherein determining said APE1 catalytic activity is effected using an oligonucleotide substrate having a furanyl abasic site lesion (AP) and wherein determining said OGG1 catalytic activity is effected using an oligonucleotide substrate having an 8-oxoguanine lesion.

12. The method of claim 11, wherein said double stranded oligonucleotide substrate having a hypoxanthine lesion (Hx) comprises an oligonucleotide sequence as set forth in SEQ ID NO: 1 or SEQ ID NO: 7 annealed to an oligonucleotide sequence as set forth in SEQ ID NO: 2 and said oligonucleotide substrate having an N6-ethenoadenine lesion (eA) comprises an oligonucleotide sequence as set forth in SEQ ID NO: 3 annealed to an oligonucleotide sequence as set forth in SEQ ID NO: 4, wherein said oligonucleotide substrate having a furanyl abasic site lesion (AP) comprises an oligonucleotide sequence as set forth in SEQ ID NO: 8 or SEQ ID NO: 10 annealed to an oligonucleotide sequence as set forth in SEQ ID NO: 9 and wherein said oligonucleotide substrate having an 8-oxoguanine lesion an oligonucleotide sequence as set forth in SEQ ID NO: 5 annealed to an oligonucleotide sequence as set forth in SEQ ID NO: 6.

13. The use of an assay kit comprising reagents for determining a level of MPG enzyme activity and at least one of APE1 and OGG1 enzyme activities in a biological sample of said subject for determining a lung cancer risk of said subject.

14. The use of claim 13, wherein said reagent for determining MPG enzyme activity comprises a double stranded oligonucleotide substrate having a hypoxanthine lesion (Hx) comprising an oligonucleotide sequence as set forth in SEQ ID NO: 1 or SEQ ID NO: 7 annealed to an oligonucleotide sequence as set forth in SEQ ID NO: 2 or an oligonucleotide substrate having an N6-ethenoadenine lesion (eA) comprises an oligonucleotide sequence as set forth in SEQ ID NO: 3 annealed to an oligonucleotide sequence as set forth in SEQ ID NO: 4; wherein said reagent for determining APE1 enzyme activity comprises an oligonucleotide substrate having a furanyl abasic site lesion (AP) comprising an oligonucleotide sequence as set forth in SEQ ID NO: 8 or SEQ ID NO: 10 annealed to an oligonucleotide sequence as set forth in SEQ ID NO: 9 and wherein said reagent for determining OGG1 enzyme activity comprises an oligonucleotide substrate having an 8-oxoguanine lesion comprising an oligonucleotide sequence as set forth in SEQ ID NO: 5 annealed to an oligonucleotide sequence as set forth in SEQ ID NO: 6.

15. The method or use of any one of claims 1-14, wherein said biological sample comprises isolated peripheral blood mononuclear cells.

16. A method of determining response of a human subject to a lung cancer treatment by a DNA damaging agent, the method comprising:
(a) determining a level of catalytic activity of N-methylpurine DNA glycosylase (MPG) in a biological sample of the subject, and, according to said level,
(b) determining the appropriate treatment and treatment regimen for treating said subject, wherein a level of said catalytic activity above a predetermined value is indicative of an increased response of said subject to a lung cancer treatment by a DNA damaging agent, and
(c) referring said subject for treatment according to said treatment regimen.

17. The method of claim 16, further comprising determining a level of catalytic activity of apurinic/apyrimidinic endonuclease 1 (APE1) or 8-oxoguanine DNA glycosylase (OGG1) or both APE1 and OGG1 in said biological sample, wherein a level of said catalytic activity of MPG in said sample of the subject above a first predetermined value, and a level of APE1 in said sample of the subject below a second predetermined value or a level of OGG1 in said sample of the subject below a third predetermined value or a level of both APE1 below said second predetermined value and a level of OGG1 below said third predetermined value is indicative of an increased responsiveness of said subject to a lung cancer treatment by a DNA damaging agent.

18. The method of any one of claims 1-12 and 15-17 or use of any one of claims 13-15, wherein said lung cancer is non-small cell lung cancer.

19. A method of selecting, from within a population of subjects, a subpopulation of subjects for referral for a lung cancer diagnostic test, the method comprising identifying a subpopulation of subjects within said population having an increased risk of developing lung cancer according to the methods of any one of claims 1-12, thereby identifying the subpopulation of subjects for referral for the lung cancer diagnostic test, and referring members of said subpopulation for at least one lung cancer diagnostic test.

20. The method of any one of claims 16-19, wherein said lung cancer diagnostic test is selected from the group consisting of mediastinoscopy, bronchoscopy, computerized tomography (CT), spiral (low dose) computerized tomography (LDCT), positron emission tomography (PET), magnetic resonance imaging (MRI), X-ray, sputum cell cytology analysis, lung biopsy, genetic profiling and lung cancer biomarker analysis.

## Patentansprüche

1. Verfahren zum Bestimmen eines Risikos eines menschlichen Probanden, Lungenkrebs zu entwickeln, wobei das Verfahren das Bestimmen eines Werts einer katalytischen Aktivität von N-Methylpurin-DNA-Glycosylase (MPG) in einer biologischen Probe des Probanden umfasst und
gemäß diesem Wert, das Bestimmen des Risikos des Probanden, Lungenkrebs zu entwickeln, wobei ein Wert der katalytischen Aktivität der MPG über einem ersten vorbestimmten Wert ein Anzeichen eines erhöhten Risikos des Probanden ist, Lungenkrebs zu entwickeln.

2. Verfahren nach Anspruch 1, ferner umfassend
(a) das Bestimmen des Werts der katalytischen Aktivität von
(i) Apurin-/Apyrimidinendonuklease 1 (APE1) oder
(ii) 8-Oxoguanin-DNA-Glycosylase (OGG1) oder
(iii) sowohl Apurin-/Apyrimidinendonuklease 1 (APE1) als auch 8-Oxoguanin-DNA-Glycosylase (OGG1)
in der biologischen Probe, und;
gemäß dem Wert, das Bestimmen des Risikos des Probanden, Lungenkrebs zu entwickeln, wobei ein Wert der katalytischen Aktivität von MPG über einem ersten vorbestimmen Wert und entweder einem Wert der katalytischen Aktivität von APE1 unter einem zweiten vorbestimmten Wert oder ein Wert der katalytischen Aktivität von OGG1 unter einem dritten vorbestimmten Wert oder sowohl ein Wert der katalytischen Aktivität von APE1 unter einem zweiten vorbestimmten Wert als auch ein Wert der katalytischen Aktivität von OGG1 unter einem dritten vorbestimmten Wert
jeweils Anzeichen eines erhöhten Risikos des Probanden sind, Lungenkrebs zu entwickeln.

3. Verfahren nach Anspruch 1, ferner umfassend:
das Bestimmen des Werts der katalytischen Aktivität wenigstens eines von Apurin-/Apyrimidinendonuklease 1 (APE1) und 8-Oxoguanin-DNA-Glycosylase (OGG1) in der biologischen Probe des Probanden;
(b) das Bestimmen einer integrierten DNA-Regenerationsbewertung für den Probanden von dem MPG-Wert und wenigstens einem von OGG1 und APE1; und
(c) das Bestimmen des Risikos des Probanden, Lungenkrebs zu entwickeln, wobei eine integrierte DNA-Regenerationsbewertung unter einem vorbestimmten Wert ein Anzeichen auf ein erhöhtes Risiko des Probanden, Lungenkrebs zu entwickeln.

4. Verfahren nach Anspruch 2 oder 3, wobei das Bestimmen der katalytischen Aktivität in (a) das Bestimmen eines Werts der katalytischen Aktivität von N-Methylpurin-DNA-Glycosylase (MPG) und von Apurin-/Apyrimidinendonuklease 1 (APE1) in der biologischen Probe des Probanden umfasst.

5. Verfahren nach Anspruch 2 oder 3, wobei das Bestimmen der katalytischen Aktivität in (a) das Bestimmen eines Werts der katalytischen Aktivität von N-Methylpurin-DNA-Glycosylase (MPG) und 8-Oxoguanin-DNA-Glycosylase (OGG1) in der biologischen Probe des Probanden umfasst.

6. Verfahren nach Anspruch 2 oder 3, wobei das Bestimmen der katalytischen Aktivität in (a) das Bestimmen eines Werts der katalytischen Aktivität von N-Methylpurin-DNA-Glycosylase (MPG), Apurin-/Apyrimidinendonuklease 1 (APE1) und von 8-Oxoguanin-DNA-Glycosylase (OGG1) in der biologischen Probe des Probanden umfasst.

7. Verfahren nach einem der Ansprüche 1 - 6, wobei das Risiko oder der Risikowert als ein Wahrscheinlichkeitsverhältnis (OR) im Vergleich zu dem Risiko der Entwicklung von Lungenkrebs desjenigen einer Referenzpopulation normaler, scheinbar gesunder Menschen ausgedrückt wird, das dem oder den Probanden für wenigstens einen Parameter zugeordnet wird, der ausgewählt ist aus der Gruppe bestehend aus Geschlecht, Alter, Ethnizität und Status des Rauchens.

8. Verfahren nach Anspruch 7, wobei das Wahrscheinlichkeitsverhältnis für die katalytische MPG-Aktivität, wenn durch den MPG-Hx-Test bestimmt, 1,18 für alle 10 Einheiten an katalytischer Aktivität oder 1,8 für jeden 1 SD über dem vorbestimmten Wert beträgt.

9. Verfahren nach Anspruch 7, wobei das kombinierte Wahrscheinlichkeitsverhältnis für die katalytischen MPG- und APE1-Aktivitäten relativ zu denen der Referenzpopulation wenigstens 5 beträgt, wobei das Wahrscheinlichkeitsverhältnis für APE1 durch Vergleichen der katalytischen APE1-Aktivität an dem 25. Perzentil mit denjenigen des 75. Perzentils der Kontrollwerte bestimmt wird und das Wahrscheinlichkeitsverhältnis für MPG durch Vergleichen der katalytischen MPG-Aktivität an dem 75. Perzentil mit denjenigen des 25. Perzentils der Kontrollwerte bestimmt wird.

10. Verfahren nach Anspruch 7, wbbei das Wahrscheinlichkeitsverhältnis aus einer integrierten DNA-Regenerationsbewertung für die kombinierte katalytische MPG-, OGG1-und APE1-Aktivität berechnet wird, wobei die integrierte DNA-Regenerationsbewertung unter dem Durchschnitt einer Referenzpopulation liegt, wobei das Wahrscheinlichkeitsverhältnis wenigstens 3,0 beträgt.

11. Verfahren nach einem der Ansprüche 1 - 10, wobei das Bestimmen der katalytischen MPG-Aktivität unter Verwendung eines doppelsträngigen Oligonukleotidsubstrats bewirkt wird, das eine Hypoxanthinläsion (Hx) aufweist oder eines Oligonukleotidsubstrats, das eine N6-Ethenadeninläsion (eA) aufweist, wobei das Bestimmen der katalytischen APE1-Aktivität unter Verwendung eines Oligonukleotidsubstrats bewirkt wird, das eine nicht funktionsfähige Furanylstellenläsion (AP) aufweist und wobei das Bestimmen der katalytischen OGG1-Aktivität unter Verwendung eines Oligonukleotidsubstrats bewirkt wird, die eine 8-Oxoguaninläsion aufweist.

12. Verfahren nach Anspruch 11, wobei das doppelsträngige Substrat, das eine Hypoxanthinläsion (Hx) aufweist, eine Oligonukleotidsequenz, wie in SEQ ID NO: 1 oder SEQ ID NO: 7 dargelegt, aufweist, die an eine Oligonukleotidsequenz, wie in SEQ ID NO: 2 dargelegt, aufgeschmolzen ist und das Oligonukleotidsubstrat, das eine N6-Ethanadeninläsion (eA) aufweist, eine Oligonukleotidsequenz, wie in SEQ ID NO: 3 dargelegt, umfasst, die an eine Oligonukleotidsequenz, wie in SEQ ID NO: 4 dargelegt, aufgeschmolzen ist, wobei das Oligonukleotidsubstrat, das eine nicht funktionsfähige Furanylstellenläsion (AP) aufweist, eine Nukleotidsequenz, wie in SEQ ID NO: 8 oder SEQ ID NO: 10 dargelegt, umfasst, die an eine Oligonukleotidsequenz, wie in SEQ ID NO: 9 dargelegt, aufgeschmolzen ist und wobei das Oligonukleotidsubstrat, das eine 8-Oxoguaninläsion aufweist, wie in SEQ ID NO: 5 dargelegt, umfasst, die an eine Oligonukleotidsequenz, wie in SEQ ID NO: 6 dargelegt, aufgeschmolzen ist.

13. Verwendung eines Test-Kits, umfassend Reagenzien zum Bestimmen eines Werts der MPG-Enzymaktivität und wenigstens einer der APE1- und OGG1-Enzymaktivitäten in einer biologischen Probe eines Probanden zum Bestimmen eines Risikos für Lungenkrebs für den Probanden.

14. Verwendung nach Anspruch 13, wobei das Reagens zum Bestimmen der MPG-Enzymaktivität ein doppelsträngiges Oligonukleotidsubstrat umfasst, das eine Hypoxanthinläsion (Hx) aufweist, umfassend eine Oligonukleotidsequenz, wie in SEQ ID NO: 1 oder SEQ ID NO: 7 dargelegt, die an eine Oligonukleotidsequenz, wie in SEQ ID NO: 2 dargelegt, aufgeschmolzen ist oder ein Oligonukleotidsubstrat, das eine N6-Ethenadeninläsion (eA) aufweist, umfassend eine Oligonukleotidsequenz, wie in SEQ ID NO: 3 dargelegt, die an eine Oligonukleotidsequenz, wie in SEQ ID NO: 4 dargelegt, aufgeschmolzen ist, wobei das Reagens zum Bestimmen der APE1-Enzymaktivität ein Oligonukleotidsubstrat, das eine nicht funktionsfähige Furanylstellenläsion (AP) aufweist, eine Nukleotidsequenz, wie in SEQ ID NO: 8 oder SEQ ID NO: 10 dargelegt, umfasst, die an eine Oligonukleotidsequenz, wie in SEQ ID NO: 9 dargelegt, aufgeschmolzen ist und wobei das Reagens zum Bestimmen der OGG1-Enzymaktivität Oligonukleotidsubstrat, das eine 8-Oxoguaninläsion aufweist, wie in SEQ ID NO: 5 dargelegt, umfasst, die an eine Oligonukleotidsequenz, wie in SEQ ID NO: 6 dargelegt, aufgeschmolzen ist.

15. Verfahren oder Verwendung nach einem der Ansprüche 1 - 14, wobei die biologische Probe isolierte periphere mononukleare Blutzellen umfasst.

16. Verfahren zum Bestimmen einer Antwort eines menschlichen Probanden auf eine Lungenkrebsbehandlung mit einem DNA-Beschädigungsmittel, wobei das Verfahren Folgendes umfasst:
(a) das Bestimmen eines Werts der katalytischen Aktivität von N-Methylpurin-DNA-Glycoxylase (MPG) in einer biologischen Probe des Probanden und gemäß diesem Wert
(b) das Bestimmen der geeigneten Behandlung und des Behandlungsplans zur Behandlung des Probanden, wobei ein Wert der katalytischen Aktivität über einem vorbestimmten Wert ein Anzeichen auf eine erhöhte Antwort des Probanden auf eine Lungenkrebsbehandlung mit einem DNA-Beschädigungsmittel ist, und
(c) das Überweisen des Probanden für eine Behandlung gemäß dem Behandlungsplan.

17. Verfahren nach Anspruch 16, ferner umfassend das Bestimmen eines Werts der katalytischen Aktivität Apurin-/Apyrimidinendonuklease 1 (APE1) oder 8-Oxoguanin-DNA-Glycosylase (OGG1) oder sowohl Apurin-/Apyrimidinendonuklease 1 (APE1) als auch 8-Oxoguanin-DNA-Glycosylase (OGG1) in der biologischen Probe, wobei ein Wert der katalytischen Aktivität von MPG in der Probe des Probanden über einem ersten vorbestimmten Wert und ein Wert von APE1 in der Probe des Probanden unter einem zweiten vorbestimmten Wert oder ein Wert von OGG1 in der Probe des Probanden unter einem dritten vorbestimmten Wert oder ein Wert von sowohl APE1 unter dem zweiten vorbestimmten Wert als auch ein Wert von OGG1 unter dem dritten vorbestimmten Wert ein Anzeichen auf ein erhöhtes Ansprechen des Probanden auf eine Lungenkrebsbehandlung mit einem DNA-Beschädigungsmittel ist.

18. Verfahren nach einem der Ansprüche 1 - 12 und 15 - 17 zur Verwendung nach einem der Ansprüche 13 - 15, wobei es sich bei dem Lungenkrebs um ein nichtkleinzelliges Bronchialkarzinom handelt.

19. Verfahren zur Auswahl, innerhalb einer Population von Probanden, einer Subpopulation von Probanden zum Überweisen für einen diagnostischen Lungenkrebstest, wobei das Verfahren das Identifizieren einer Subpopulation von Probanden innerhalb der Population mit einem erhöhten Risiko zur Entwicklung von Lungenkrebs gemäß den Verfahren nach einem der Ansprüche 1 - 12 umfasst, wodurch die Subpopulation von Probanden zum Überweisen für den diagnostischen Lungenkrebstest identifiziert wird und Mitglieder der Subpopulation für wenigstens einen diagnostischen Lungenkrebstest überwiesen werden.

20. Verfahren nach einem der Ansprüche 16 - 19, wobei der diagnostische Lungenkrebstest ausgewählt ist aus der Gruppe bestehend aus Mediastinoskopie, Bronchoskopie, Computertomographie (CT), spirale (Niedrigdosis-) Computertomographie (LDCT), Positronen-Emissions-Tomographie (PET), Magnetresonanztomographie (MRI), Röntgen, Sputum-Zellzytologieanalyse, Lungenbiopsie, genetische Profilierung und Lungenkrebs-Biomarkeranalyse.

## Revendications

1. Procédé de détermination d'un risque d'un sujet humain de développer le cancer du poumon, le procédé comprenant la détermination d'un niveau d'activité catalytique de la N-méthyl-purine ADN glycosylase (MPG) dans un échantillon biologique du sujet, et,
selon ledit niveau, la détermination du risque du sujet de développer le cancer du poumon, où un niveau de ladite activité catalytique de la MPG au-dessus d'une première valeur prédéterminée est indicatif d'un risque accru dudit sujet de développer le cancer du poumon.

2. Procédé selon la revendication 1, comprenant en outre (a) la détermination d'un niveau d'activité catalytique de
(i) l'endonucléase 1 apurinique/apyrimidinique (APE1) ou
(ii) la 8-oxoguanine ADN glycosylase (OGG1) ou
(iii) à la fois l'endonucléase 1 apurinique/apyrimidinique (APE1) et la 8-oxoguanine ADN glycosylase (OGG1)
dans ledit échantillon biologique, et ;
selon ledit niveau, la détermination du risque du sujet de développer le cancer du poumon, où un niveau de ladite activité catalytique de la MPG au-dessus d'une première valeur prédéterminée et soit un niveau de ladite activité catalytique de l'APE1 en dessous d'une deuxième valeur prédéterminée, soit un niveau de ladite activité catalytique de l'OGG1 en dessous d'une troisième valeur prédéterminée, ou à la fois un niveau de ladite activité catalytique de l'APE1 en dessous de ladite deuxième valeur prédéterminée et un niveau de ladite activité catalytique de l'OGG1 en dessous de ladite troisième valeur prédéterminée
est indicatif d'un risque accru dudit sujet de développer le cancer du poumon.

3. Procédé selon la revendication 1, comprenant en outre :
la détermination du niveau d'activité catalytique d'au moins l'une de l'endonucléase 1 apurinique/apyrimidinique (APE1) et la 8-oxoguanine ADN glycosylase (OGG1) dans ledit échantillon biologique du sujet ;
(b) la détermination d'un score de réparation de l'ADN intégré pour ledit sujet à partir dudit niveau de la MPG et d'au moins l'une d'OGG1 et APE1 ; et
(c) la détermination du risque du sujet de développer le cancer du poumon, où un score de réparation de l'ADN intégré en dessous d'une valeur prédéterminée est indicatif d'un risque accru dudit sujet de développer le cancer du poumon.

4. Procédé selon la revendication 2 ou 3, dans lequel ladite détermination de ladite activité catalytique en (a) comprend la détermination d'un niveau d'activité catalytique de la N-méthyl-purine ADN glycosylase (MPG) et de l'endonucléase 1 apurinique/apyrimidinique (APE1) dans ledit échantillon biologique du sujet.

5. Procédé selon la revendication 2 ou 3, dans lequel ladite détermination de ladite activité catalytique en (a) comprend la détermination d'un niveau d'activité catalytique de la N-méthyl-purine ADN glycosylase (MPG) et la 8-oxoguanine ADN glycosylase (OGG1) dans ledit échantillon biologique du sujet.

6. Procédé selon la revendication 2 ou 3, dans lequel ladite détermination de ladite activité catalytique en (a) comprend la détermination d'un niveau d'activité catalytique de la N-méthyl-purine ADN glycosylase (MPG), de l'endonucléase 1 apurinique/apyrimidinique (APE1) et de la 8-oxoguanine ADN glycosylase (OGG1) dans ledit échantillon biologique du sujet.

7. Procédé selon l'une quelconque des revendications 1 à 6, dans lequel ledit risque ou niveau de risque est exprimé en tant que rapport des cotes (OR) par comparaison au risque de développer le cancer du poumon de celui d'une population de référence constituée d'individus normaux apparemment en bonne santé correspondant audit/auxdits sujet ou sujets pour au moins un paramètre sélectionné dans le groupe consistant en le sexe, l'âge, l'ethnicité et le statut tabagique.

8. Procédé selon la revendication 7, dans lequel le rapport des cotes pour l'activité catalytique de la MPG, lorsqu'il est déterminé par l'essai MPG-Hx, est de 1,18 pour chaque 10 unités d'activité catalytique ou de 1,8 pour chaque 1 EC au-dessus de ladite valeur prédéterminée.

9. Procédé selon la revendication 7, dans lequel le rapport des cotes combiné pour les activités catalytiques de la MPG et l'APE1, par rapport à celui de ladite population de référence, est d'au moins 5, où le rapport des cotes pour l'APE1 est déterminé en comparant l'activité catalytique de l'APE1 au 25^{e} percentile avec celles du 75^{e} percentile des valeurs de contrôle et le rapport des cotes pour la MPG est déterminé en comparant l'activité catalytique de la MPG au 75^{e} percentile avec celles du 25^{e} percentile des valeurs de contrôle.

10. Procédé selon la revendication 7, dans lequel le rapport des cotes est calculé à partir d'un score de réparation de l'ADN intégré pour l'activité catalytique de la MPG, l'OGG1 et l'APE1 combinée, où lorsque ledit score de réparation de l'ADN intégré est en dessous de la médiane d'une population de référence, ledit rapport des cotes est au moins 3,0.

11. Procédé selon l'une quelconque des revendications 1 à 10, dans lequel la détermination de ladite activité catalytique de la MPG est effectuée en utilisant un substrat oligonucléotidique double brin comportant une lésion hypoxanthine (Hx) ou en utilisant un substrat oligonucléotidique comportant une lésion N6-éthénoadénine (eA), dans lequel la détermination de ladite activité catalytique de l'APE1 est effectuée en utilisant un substrat oligonucléotidique comportant une lésion de site abasique furanyle (AP) et dans lequel la détermination de ladite activité catalytique de l'OGG1 est effectuée en utilisant un substrat oligonucléotidique comportant une lésion 8-oxoguanine.

12. Procédé selon la revendication 11, dans lequel ledit substrat oligonucléotidique double brin comportant une lésion hypoxanthine (Hx) comprend une séquence oligonucléotidique telle que décrite dans SEQ ID NO: 1 ou SEQ ID NO: 7 annelée à une séquence oligonucléotidique telle que décrite dans SEQ ID NO: 2 et ledit substrat oligonucléotidique comportant une lésion N6-éthénoadénine (eA) comprend une séquence oligonucléotidique telle que décrite dans SEQ ID NO: 3 annelée à une séquence oligonucléotidique telle que décrite dans SEQ ID NO: 4, où ledit substrat oligonucléotidique comportant une lésion de site abasique furanyle (AP) comprend une séquence oligonucléotidique telle que décrite dans SEQ ID NO: 8 ou SEQ ID NO: 10 annelée à une séquence oligonucléotidique telle que décrite dans SEQ ID NO: 9 et où ledit substrat oligonucléotidique comportant une lésion 8-oxoguanine comprend une séquence oligonucléotidique telle que décrite dans SEQ ID NO: 5 annelée à une séquence oligonucléotidique telle que décrite dans SEQ ID NO: 6.

13. Utilisation d'un kit de test comprenant des réactifs pour déterminer un niveau d'activité enzymatique de la MPG et au moins l'une des activités enzymatiques de l'APE1 et l'OGG1 dans un échantillon biologique dudit sujet afin de déterminer un risque de cancer du poumon dudit sujet.

14. Utilisation selon la revendication 13, dans laquelle ledit réactif pour déterminer l'activité enzymatique de la MPG comprend un substrat oligonucléotidique double brin comportant une lésion hypoxanthine (Hx) comprenant une séquence oligonucléotidique telle que décrite dans SEQ ID NO: 1 ou SEQ ID NO: 7 annelée à une séquence oligonucléotidique telle que décrite dans SEQ ID NO: 2 ou un substrat oligonucléotidique comportant une lésion N6-éthénoadénine (eA) comprenant une séquence oligonucléotidique telle que décrite dans SEQ ID NO: 3 annelée à une séquence oligonucléotidique telle que décrite dans SEQ ID NO: 4 ; dans laquelle ledit réactif pour déterminer l'activité enzymatique de l'APE1 comprend un substrat oligonucléotidique comportant une lésion dé site abasique furanyle (AP) comprenant une séquence oligonucléotidique telle que décrite dans SEQ ID NO: 8 ou SEQ ID NO: 10 annelée à une séquence oligonucléotidique telle que décrite dans SEQ ID NO: 9 et dans laquelle ledit réactif pour déterminer l'activité enzymatique de l'OGG1 comprend un substrat oligonucléotidique comportant une lésion 8-oxoguanine comprenant une séquence oligonucléotidique telle que décrite dans SEQ ID NO: 5 annelée à une séquence oligonucléotidique telle que décrite dans SEQ ID NO: 6.

15. Procédé ou utilisation selon l'une quelconque des revendications 1 à 14, où ledit échantillon biologique comprend des cellules mononucléaires du sang périphérique isolées.

16. Procédé de détermination de la réponse d'un sujet humain à un traitement du cancer du poumon par un agent endommageant l'ADN, le procédé comprenant :
(a) la détermination d'un niveau d'activité catalytique de la N-méthyl-purine ADN glycosylase (MPG) dans un échantillon biologique du sujet, et, selon ledit niveau,
(b) la détermination du traitement et du régime thérapeutique appropriés pour traiter ledit sujet, où un niveau de ladite activité catalytique au-dessus d'une valeur prédéterminée est indicatif d'une réponse accrue dudit sujet à un traitement du cancer du poumon par un agent endommageant l'ADN, et
(c) l'orientation dudit sujet pour recevoir un traitement selon ledit régime thérapeutique.

17. Procédé selon la revendication 16, comprenant en outre la détermination d'un niveau d'activité catalytique de l'endonucléase 1 apurinique/apyrimidinique (APE1) ou de la 8-oxoguanine ADN glycosylase (OGG1), ou à la fois de l'APE1 et de l'OGG1 dans ledit échantillon biologique, dans lequel un niveau de ladite activité catalytique de la MPG dans ledit échantillon du sujet au-dessus d'une première valeur prédéterminée, et un niveau de l'APE1 dans ledit échantillon du sujet en dessous d'une deuxième valeur prédéterminée ou un niveau de l'OGG1 dans ledit échantillon du sujet en dessous d'une troisième valeur prédéterminée, ou un niveau d'à la fois l'APE1 en dessous de ladite deuxième valeur prédéterminée et un niveau de l'OGG1 en dessous de ladite troisième valeur prédéterminée est indicatif d'une réponse accrue dudit sujet à un traitement du cancer du poumon par un agent endommageant l'ADN.

18. Procédé selon l'une quelconque des revendications 1 à 12 et 15 à 17 ou utilisation selon l'une quelconque des revendications 13 à 15, où ledit cancer du poumon est un cancer du poumon non à petites cellules.

19. Procédé de sélection, au sein d'une population de sujets, d'une sous-population de sujets à des fins d'orientation pour effectuer un test diagnostique du cancer du poumon, le procédé comprenant l'identification d'une sous-population de sujets au sein de ladite population présentant un risque accru de développer le cancer du poumon selon les procédés de l'une quelconque des revendications 1 à 12, en identifiant ainsi la sous-population de sujets à des fins d'orientation pour effectuer le test diagnostique du cancer du poumon, et l'orientation des membres de ladite sous-population pour effectuer au moins un test diagnostique du cancer du poumon.

20. Procédé selon l'une quelconque des revendications 16 à 19, dans lequel ledit test diagnostique du cancer du poumon est sélectionné dans le groupe consistant en la médiastinoscopie, la bronchoscopie, la tomodensitométrie (TDM), la tomodensitométrie spiralée (faible dose) (TDMS), la tomographie par émission de positons (TEP), l'imagerie par résonance magnétique (IRM), la radiographie, une analyse cytologique de cellules d'expectorations, une biopsie pulmonaire, un profilage génétique et une analyse de biomarqueurs du cancer du poumon.
